(19) Europäisches Patentamt
European Patent Office
Office européen des brevets

(11) **EP 4 260 740 A1**

(12) **EUROPEAN PATENT APPLICATION**
published in accordance with Art. 153(4) EPC

(43) Date of publication:
**18.10.2023 Bulletin 2023/42**

(21) Application number: **20965834.3**

(22) Date of filing: **14.12.2020**

(51) International Patent Classification (IPC):
**A24F 40/65** [(2020.01)]

(52) Cooperative Patent Classification (CPC):
**A24F 40/65**

(86) International application number:
**PCT/JP2020/046516**

(87) International publication number:
**WO 2022/130446 (23.06.2022 Gazette 2022/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(71) Applicant: **Japan Tobacco Inc.**
**Tokyo 105-6927 (JP)**

(72) Inventors:
• **KATO, Masato**
**Tokyo 130-8603 (JP)**
• **SHIMADA, Yurina**
**Tokyo 130-8603 (JP)**

(74) Representative: **Hoffmann Eitle**
**Patent- und Rechtsanwälte PartmbB**
**Arabellastraße 30**
**81925 München (DE)**

(54) **INFORMATION PROCESSING DEVICE, INFORMATION PROCESSING METHOD, AND INFORMATION PROCESSING SYSTEM**

(57) An objective is to provide a system that broadly visualizes the status of the transition to an aerosol-generating device by a user, and assists with the transition effectively. An information processing device according to the present disclosure includes: an input receiver that receives input of goal information for transitioning from a use of a first inhalation product to a use of a second inhalation product and input of usage information about the first inhalation product; an acquirer that acquires usage information about the second inhalation product from the second inhalation product; a goal determiner that determines a goal value for each predetermined period on the basis of the goal information; a history determiner that determines usage history information for each predetermined period on the basis of the usage information about the first inhalation product and the usage information about the second inhalation product; a progress determiner that determines progress information for each predetermined period with respect to the goal value on the basis of the goal value and the usage history information; and an output unit that creates and outputs a progress screen on the basis of the progress information.

Fig. 6

EP 4 260 740 A1

**Description**

TECHNICAL FIELD

The present disclosure relates to an information processing device, an information processing method, and an information processing system.

BACKGROUND ART

Inhalation products with which a user inhales flavors are widely known. Such inhalation products include aerosol-generating devices or flavor inhalers. An aerosol-generating device refers to an electronic device for inhaling a generated aerosol, including but not limited to electronic tobacco products, heated tobacco products, and medical nebulizers, for example. Moreover, the collective term flavor inhaler is not limited to only the aerosol-generating device described above, but also includes traditional cigarettes, for example.

In particular, electronic tobacco products and heated tobacco products are becoming more widespread as products that can potentially reduce the risks to user health associated with smoking from the standpoint of nicotine dependency compared to traditional cigarettes that involve combustion. Such products that can potentially reduce the risks to user health associated with smoking may be referred to as reduced-risk products (RRPs).

In this context, technologies are in development for encouraging users to use electronic tobacco products and heated tobacco products in particular among inhalation products. Patent Literature 1 discloses a technology of the related art that acquires consumption quantities of a plurality of aerosol-generating products to display a conversion ratio related to the conversion of consumption from one to another.

CITATION LIST

PATENT LITERATURE

Patent Literature 1: International Publication No. WO 2018/060798

SUMMARY OF INVENTION

TECHNICAL PROBLEM

An objective of the present disclosure is to provide a system that broadly visualizes the status of the transition to an aerosol-generating device by a user, and assists the user with the transition effectively. More specifically, an objective of the present disclosure is to provide a system that, by collecting and processing various data regarding a user's intentions and history in relation to the transition to an aerosol-generating device, can visualize and present the progress and result regarding the transition to the user in an appropriate format.

SOLUTION TO PROBLEM

To address the above problem, in a first aspect, an information processing device is provided. The information processing device includes: an input receiver that receives input of goal information for transitioning from a use of a first inhalation product to a use of a second inhalation product and input of usage information about the first inhalation product; an acquirer that acquires usage information about the second inhalation product from the second inhalation product; a goal determiner that determines a goal value for each predetermined period on the basis of the goal information; a history determiner that determines usage history information for each predetermined period on the basis of the usage information about the first inhalation product and the usage information about the second inhalation product; a progress determiner that determines progress information for each predetermined period with respect to the goal value on the basis of the goal value and the usage history information; and an output unit that creates and outputs a progress screen on the basis of the progress information.

According to an information processing device of a second aspect, in the information processing device of the first aspect, the input receiver is further configured to receive input for editing the goal information, and the goal determiner is further configured to update the determined goal value on the basis of the edited goal information.

According to an information processing device of a third aspect, in the information processing device of the first or second aspect, the input receiver is further configured to receive the input of usage information about the first inhalation product through a prescribed user interface image every time a user uses the first inhalation product, and the usage information about the first inhalation product is associated with a time at which the usage information about the first inhalation product is received.

According to an information processing device of a fourth aspect, the information processing device of any of the first to third aspects further includes a reader for reading a prescribed code, the usage information about the first inhalation product includes a brand selected from among a plurality of brands applicable to the first inhalation product, and the brand is specified automatically by causing the reader to read the code included in the first inhalation product.

According to an information processing device of a fifth aspect, in the information processing device of any of the first to fourth aspects, the goal information includes a deadline, and the goal determiner is configured to determine an ultimate goal value on the deadline on the basis of the goal information and determine the goal value for each predetermined period until the deadline on the basis of the ultimate goal value.

According to an information processing device of a sixth aspect, in the information processing device of the fifth aspect, the predetermined period is set to one day, and the daily goal value includes at least one of a daily planned usage value of the first inhalation product or a daily planned usage value of the second inhalation product calculated on the basis of the ultimate goal value and the number of days until the day of the deadline.

According to an information processing device of a seventh aspect, in the information processing device of the sixth aspect, the progress information includes a daily score, the progress determiner is configured to determine a daily usage ratio of the second inhalation product on the basis of the usage history information and calculate the daily score on the basis of a ratio of the daily planned usage value of the second inhalation product and the daily usage ratio of the second inhalation product, and the progress screen includes a display of the score.

According to an information processing device of an eighth aspect, in the information processing device of the seventh aspect, the progress information includes a daily achievement result ranked on the basis of the daily score, and the progress screen includes a display of a calendar with which the achievement result is visually associated.

According to an information processing device of a ninth aspect, in the information processing device of any of the sixth to eighth aspects, the progress information includes a value of a prescribed indicator calculated on the basis of at least one of the usage information about the first inhalation product and the usage information about the second inhalation product, and the progress screen includes a display of a graph illustrating how the value of the prescribed indicator changes daily over time.

According to an information processing device of a 10th aspect, in the information processing device of any of the first to ninth aspects, the first inhalation product is a cigarette, and the second inhalation product is an aerosol-generating device that can communicate with the information processing device.

In an 11th aspect, an information processing method is provided. The information processing method includes: receiving input of goal information for transitioning from a use of a first inhalation product to a use of a second inhalation product; determining a goal value for each predetermined period until a deadline on the basis of the goal information; receiving input of usage information about the first inhalation product; acquiring usage information about the second inhalation product from the second inhalation product; determining usage history information for each predetermined period on the basis of the usage information about the first inhalation product and the usage information about the second inhalation product; determining progress information for each predetermined period with respect to the goal value on the basis of the goal value and the usage history information; and creating and outputting a progress screen based on the progress information.

According to an information processing method of a 12th aspect, the information processing method of the 11th aspect further includes: receiving input for editing the goal information; and updating the goal value on the basis of the edited goal information.

According to an information processing method of a 13th aspect, in the information processing method of the 11th or 12th aspect, the usage information about the first inhalation product is configured to be inputted through a prescribed user interface image every time a user uses the first inhalation product, and the usage information about the second inhalation product is configured to be acquired from the second inhalation product through communication.

According to an information processing method of a 14th aspect, in the information processing method of any of the 11th to 13th aspects, the goal information includes a deadline, and the determining the goal value includes: determining an ultimate goal value on the deadline on the basis of the goal information; and determining the goal value for each predetermined period until the deadline on the basis of the ultimate goal value.

According to an information processing method of a 15th aspect, in the information processing method of the 14th aspect, the predetermined period is set to one day, and the goal value includes at least one of a daily planned usage value of the first inhalation product or a daily planned usage value of the second inhalation product calculated on the basis of the ultimate goal value and the number of days until the day of the deadline.

According to an information processing method of a 16th aspect, in the information processing method of the 15th aspect, the progress information includes a daily score calculated on the basis of a ratio of a daily planned usage value of the second inhalation product and a daily usage ratio of the second inhalation product, and the progress screen includes a display of the score.

According to an information processing method of a 17th aspect, in the information processing method of the 16th aspect, the progress information includes a daily achievement result ranked on the basis of the daily score, and the progress screen includes a display of a calendar with which the achievement result is visually associated.

According to an information processing method of an 18th aspect, in the information processing method of any of the 15th to 17th aspects, the progress information includes a value of a prescribed indicator calculated on the basis of at least one of the usage information about the first inhalation product and the usage information about the second inhalation product, and the progress screen includes a display of a graph illustrating how the value of the prescribed indicator changes daily over time.

In a 19th aspect, an information processing system is provided. The information processing system includes a user terminal and a server, the user terminal including: an input receiver that receives input of goal information to transition from a use of a first inhalation product to a use of a second inhalation product and input of usage information about the first inhalation product; an acquirer that acquires usage information about the second inhalation product from the second inhalation product; and an output unit that generates and outputs a progress screen based on progress information, the server including: a goal determiner that determines a goal value for each predetermined period on the basis of the goal information; a history determiner that determines usage history information for each predetermined period on the basis of the usage information about the first inhalation product and the usage information about the second inhalation product; and a progress determiner that determines the progress information for each predetermined period with respect to the goal value on the basis of the goal value and the usage history information.

According to an information processing system of a 20th aspect, in the information processing system of the 19th aspect, the server further includes a ranking determiner that determines a ranking of the user on the basis of the progress information, and the progress screen to be displayed by the output unit of the user terminal is configured to display the ranking.

BRIEF DESCRIPTION OF DRAWINGS

[Fig. 1] Fig. 1 is a schematic configuration diagram of an overall system.
[Fig. 2] Fig. 2 is a schematic diagram of a configuration example of an aerosol-generating device.
[Fig. 3] Fig. 3 is a schematic diagram of a hardware configuration example of a user terminal.
[Fig. 4] Fig. 4 is a schematic diagram of a hardware configuration example of a server.
[Fig. 5] Fig. 5 is a schematic functional configuration diagram of an information processing system according to a first embodiment.
[Fig. 6] Fig. 6 is a schematic process flowchart of the information processing system according to the first embodiment.
[Fig. 7] Fig. 7 is a detailed flowchart of some of the processing steps in Fig. 6.
[Fig. 8] Fig. 8 is a schematic transition diagram of screen images outputted to a user terminal.
[Fig. 9A] Fig. 9A is a schematic diagram of an example of an input screen image displayed on a user terminal.
[Fig. 9B] Fig. 9B is a schematic diagram of an example of an input screen image displayed on a user terminal.
[Fig. 10] Fig. 10 is a schematic diagram of an example of an output screen image displayed on a user terminal.
[Fig. 11A] Fig. 11A is a schematic diagram of an example of an input screen image displayed on a user terminal.
[Fig. 11B] Fig. 11B is a schematic diagram of an example of an input screen image displayed on a user terminal.
[Fig. 12A] Fig. 12A is a schematic diagram of an example of an output screen image displayed on a user terminal.
[Fig. 12B] Fig. 12B is a schematic diagram of an example of an output screen image displayed on a user terminal.
[Fig. 13] Fig. 13 is a schematic functional configuration diagram of an information processing system according to a second embodiment.
[Fig. 14] Fig. 14 is a schematic process flowchart of the information processing system according to the second embodiment.
[Fig. 15] Fig. 15 is an example of a ranking screen image displayed on a user terminal.

DESCRIPTION OF EMBODIMENTS

Hereinafter, an information processing device, an information processing method, and an information processing system according to embodiments of the present disclosure will be described with reference to the accompanying drawings. In the accompanying drawings, the same or similar elements are denoted with the same or similar reference signs, and duplicate descriptions of the same or similar elements may be omitted from the description of each embodiment. Also, the features indicated in each embodiment are also applicable to other embodiments insofar as the embodiments do not contradict each other. Furthermore, the drawings are schematic and do not necessarily correspond to actual dimensions, proportions, and the like. The drawings may also include portions where the dimensional relationships and ratios differ from each other.

According to the embodiments, a user can be effectively encouraged to transition from the use of a first inhalation product to the use of a second inhalation product. In the following description, an example of the first inhalation product is assumed to be a cigarette which is a non-aerosol-generating device and an example of the second inhalation product is assumed to be an aerosol-generating device (in particular, a heated flavor inhaler), but the inhalation products are not limited to the above.

<1. Overall system>

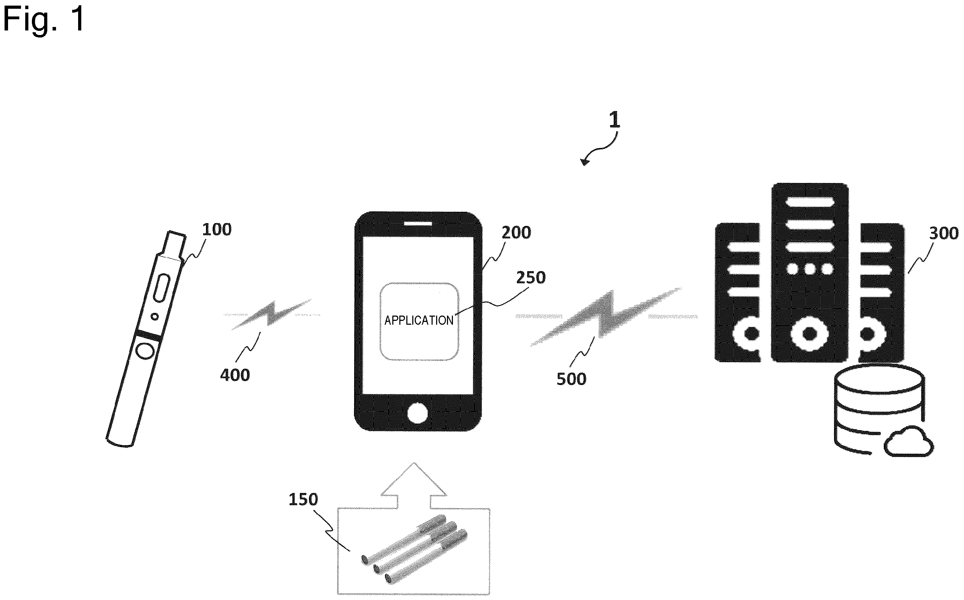

Fig. 1 is a schematic configuration diagram of an overall system 1 related to an embodiment of the present disclosure, the overall system 1 assisting a user in the transition from the use of a cigarette 150 to the use of an aerosol-generating device 100. The overall system 1 is provided with an aerosol-generating device 100, a cigarette 150, a user terminal 200, a server 300, and networks 400 and 500.

The aerosol-generating device 100 is an electronic device product that generates an aerosol or an aerosol with added flavor to be inhaled by a user. The cigarette 150 is a utensil product obtained by rolling finely cut tobacco leaf into a long, thin shape with paper. The user terminal 200 acquires various information from an external source and executes an application 250 for transitioning from the cigarette 150 to the aerosol-generating device 100. The server 300 cooperates with the application 250 on the user terminal 200 to manage various data related to the user and the application 250 and the like. Note that the server 300 may also executes some of the processes by the application 250.

The network 400 connects the aerosol-generating device 100 and the user terminal 200. The connection may be a wireless connection or a wired connection. The network 500 connects the user terminal 200 and the server 300 wirelessly, and may be the Internet in one example. Note that with regard to the cigarette 150, the user terminal 200 acquires information about the cigarette 150 through manual input or the like from the user, for example.

Hereinafter, the configuration of each of the aerosol-generating device 100, the cigarette 150, the user terminal 200, and the server 300 provided in the system 1 will be described.

<1-1. Aerosol-generating device>

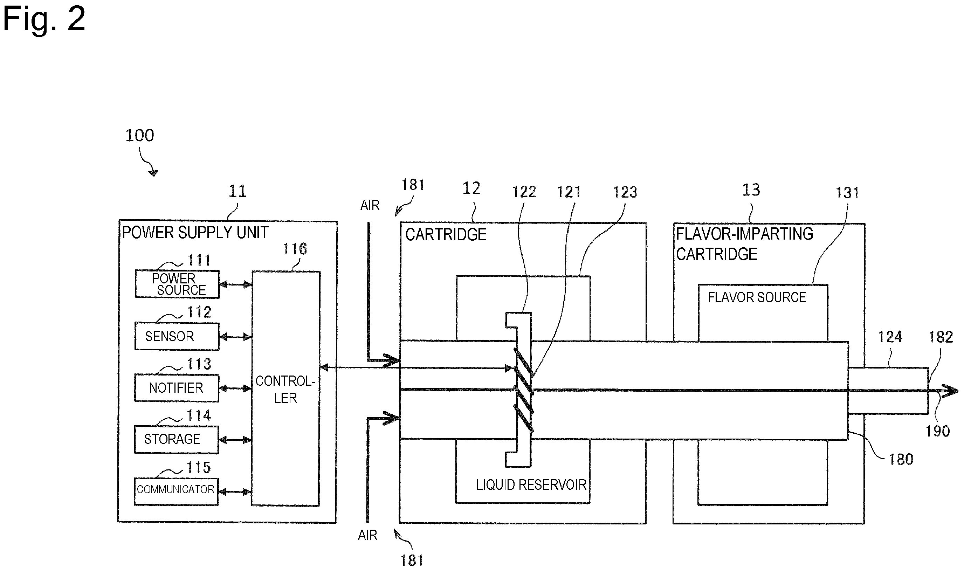

Fig. 2 is a schematic diagram of a configuration example of the aerosol-generating device 100. The aerosol-generating device 100 may be a heated flavor inhaler, for example, and may include any of various inhalation devices that generate an aerosol or an aerosol with added flavor to be inhaled by the user. Moreover, the generated inhalation component may also contain a gas such as invisible vapor in addition to an aerosol.

The aerosol-generating device 100 includes a power supply unit 11, a cartridge 12, and a flavor-imparting cartridge 13. Functionally, the power supply unit 11 includes a power source 111, a sensor 112, a notifier 113, storage 114, a communicator 115, and a controller 116. The cartridge 12 includes a heater 121, a liquid channel 122, and a liquid reservoir 123. The flavor-imparting cartridge 13 includes a flavor source 131 and a mouthpiece 124. An air channel 180 is formed in the cartridge 12 and the flavor-imparting cartridge 13.

The power source 111 stores electric power. The power source 111 supplies electric power to each component of the aerosol-generating device 100 on the basis of control by the controller 116. The power source 111 is formed from a rechargeable battery such as a lithium-ion secondary cell, for example.

The sensor 112 acquires various information related to operations by the aerosol-generating device 100. As an example, the sensor 112 may include a button, switch, or the like, and detects when the aerosol-generating device 100 is powered on or off. The sensor 112 may also include a pressure sensor such as a microphone condenser, a flow rate sensor, a temperature sensor, or the like, and detect various values (such as temperature information, battery information, and malfunction information, for example) related to inhalation operations (puff operations) by the user.

Furthermore, the sensor 112 is achieved with an inhalation sensor and acquires the number of puff operations (puff count) by the user. The sensor 112 also cooperates with a timer (not illustrated) provided in the controller 116 to acquire a puff operation time over a series of puff operations. In addition, the sensor 112 may be achieved with a contact sensor or the like, and by detecting the attachment/detachment of the flavor-imparting cartridge 13, acquire a consumption quantity (such as the number of times a cartridge is used or replaced, for example) consumed through inhalation operations by the user. Alternatively, the consumption quantity of the flavor-imparting cartridge 13 may be calculated from puff information such as the puff count of the aerosol-generating device 100. For example, a series of 15 puffs by the user may be associated with the consumption of one flavor-imparting cartridge 13.

In this way, by acquiring the puff count, the puff operation time, and/or the consumption quantity of the flavor-imparting cartridge 13, a usage history of the aerosol-generating device 100 by the user can be specified. Note that in the following, the consumption quantity of the aerosol-generating device 100 is assumed to be related to the puff count of the aerosol-generating device 100 and/or the used number of the flavor-imparting cartridges 13. However, the consumption quantity is not limited to the above and may also include the number of the cartridge 12 instead of, or in addition to, the used number of the flavor-imparting cartridges 13, for example.

Information related to puff operations by the user and information related to a usage history of the aerosol-generating device 100 may be stored in the storage 114 as usage information about the aerosol-generating device 100 together with corresponding times specified by the timer (not illustrated) provided in the controller 116.

The notifier 113 notifies the user about various information related to operations by the aerosol-generating device 100. For example, the notifier 113 is configured as a light-emitting device (for example, an LED) that emits light, a display device that displays images, a sound output device that outputs sound, a vibration device that vibrates, or the like.

The storage 114 stores various information related to operations by the aerosol-generating device 100. For example, the storage 114 includes a non-volatile storage medium such as flash memory. The storage 114 also stores programs such as firmware in addition to computer-executable instructions for causing the aerosol-generating device 100 to operate.

The communicator 115 is a communication interface capable of performing communication conforming to any given wired or wireless communication standard. For example, in the case of wireless communication, Wi-Fi(R), Bluetooth(R), or the like may be adopted. Also, in the case of wired communication, a data communication cable is connected through an external connection terminal such as Micro-USB, for example.

The communicator 115 inputs/outputs data related to operations by the aerosol-generating device 100 with respect to an external device such as the user terminal 200. Note that the communicator 115 may be provided internally in the aerosol-generating device 100, or may be provided in a communication device that can be removably attached to the aerosol-generating device 100 and function together with the aerosol-generating device.

The controller 116 functions as a computational processing device and control device, and controls overall operations inside the aerosol-generating device 100 by following various programs. The controller 116 is achieved by an electronic circuit such as a central processing unit (CPU) or a microprocessor, for example.

The heater 121 heats an aerosol source to atomize the aerosol source and generate an aerosol. For example, the heater 121 is configured as a coil that is wound around the liquid channel 122. The heater 121 generates heat when supplied with power from the power source 111. Additionally, when the heater 121 generates heat, the aerosol source held in the liquid channel 122 is heated and atomized, and an aerosol is generated.

The aerosol source, that is, the liquid stored in the liquid reservoir 123, is channeled from the liquid reservoir 123 and held in the liquid channel 122. For example, the liquid channel 122 may be a wick formed by twisting fiber materials such as glass fiber or other porous materials such as porous ceramics. In this case, the aerosol source stored in the liquid reservoir 123 is channeled by the capillary effect of the wick.

The liquid reservoir 123 stores an aerosol source. For example, the aerosol source may be a polyhydric alcohol such as glycerin or propylene glycol and a liquid such as water. The aerosol source may also contain tobacco-derived or non-tobacco-derived flavor components.

The air channel 180 is a channel for air to be inhaled by the user. The air channel 180 has a tubular structure with an air inflow hole 181 serving as an inlet for air into the air channel 180 and an air outflow hole 182 serving as an outlet for air from the air channel 180 on either end. Air flowing in from the air inflow hole 181 in association with inhalation by the user is mixed with the aerosol generated by the heater 121, and as indicated by the arrow 190, passes through the flavor source 131 and is transported to the air outflow hole 182. When the fluid mixture of aerosol and air passes through the flavor source 131, the flavor component contained in the flavor source 131 is imparted to the aerosol.

The mouthpiece 124 is a member that is put into the user's mouth during inhalation. The air outflow hole 182 is disposed in the mouthpiece 124. The user puts the mouthpiece 124 into the user's mouth and inhales, and can thereby draw in the fluid mixture of aerosol and air into the oral cavity.

The above describes a configuration example of the aerosol-generating device 100. It is understood by a person skilled in the art that the configuration of the aerosol-generating device 100 is not limited to the above.

<1-2. Cigarette>

The cigarette 150 is a disposable tobacco product obtained by rolling cut tobacco leaf in paper. For example, the cigarette 150 is a traditional filtered cigarette as illustrated in Fig. 1. A filtered cigarette typically contains cut tobacco, wrap paper, a filter, and tipping paper.

Cut tobacco is obtained by shredding and drying tobacco leaf. A unique taste is created by adjusting the blend, adding flavors, and the like according to the brand of filtered cigarette. Typically, a plurality of the cigarettes 150 are contained in a package for each brand, and every time the user smokes, one cigarette 150 is retrieved from the package and ignited, whereby the cut tobacco is combusted.

The filter filters smoke to reduce tar and nicotine. For example, an acetate filter created from acetate, a dual charcoal filter with added activated carbon, and an AFT filter having a distinctive shape are known.

The tipping paper rolls up the cut tobacco and the filter, and wood (pulp) is typically used as the raw material.

<1-3. User terminal>

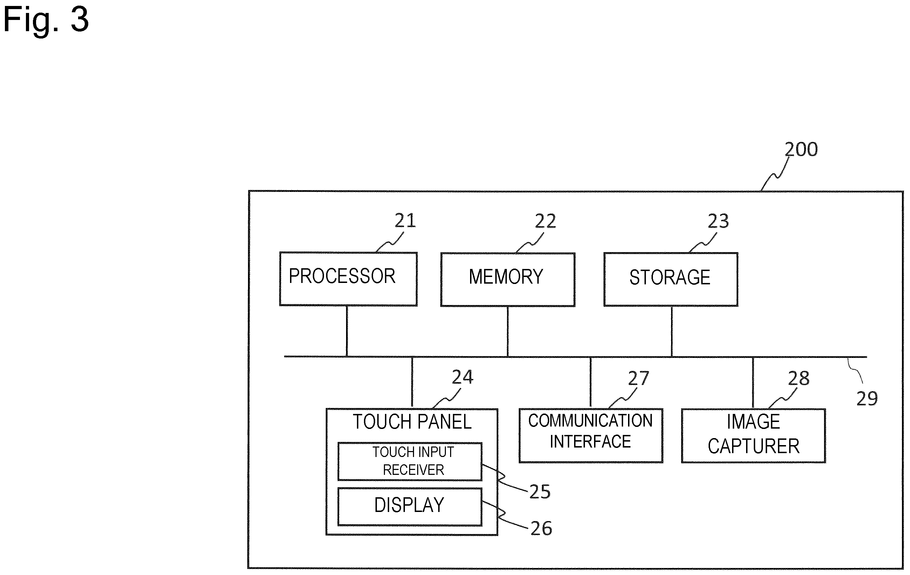

Fig. 3 is a schematic diagram of a hardware configuration example of the user terminal 200. For example, the user terminal 200 may be an electronic device such as a smartphone, a tablet terminal, a laptop computer, a personal computer, a feature phone, or a personal digital assistant (PDA). Note that a smartphone is assumed in the example of the user terminal 200 illustrated in Fig. 3.

The user terminal 200 is provided with a processor 21, a memory 22, storage 23, a touch panel 24, a communication interface 27, and an image capturer 28. These elements are electrically connected to each other via a bus 29.

The processor 21 is a computational processing device that controls information processing, controls the exchange of data between elements, and executes an information processing method according to an embodiment. For example, the processor 21 is a CPU that executes a program such as the application 250 stored in the storage 23 and loaded in the memory 22 to execute an information processing method according to an embodiment. Moreover, the processor 21 of the user terminal 200 may also be provided with a graphics processing unit (GPU) or the like in addition to a CPU.

The memory 22 includes main memory achieved with a volatile storage device such as dynamic random access memory (DRAM) and auxiliary memory achieved with a non-volatile storage device such as flash memory or a hard disk drive (HDD). The memory 22 is used as a work area for the processor 21 and the like, and stores a basic input/output system (BIOS) executed during the startup of an operating system (OS), various settings information, and the like.

The storage 23 stores programs for information processing by the processor 21, the OS, and the like. A database storing various data to be used in the execution of programs may also be constructed in the storage 23.

The touch panel 24 forms an input-output interface. The touch panel 24 includes a touch input receiver 25 and a display 26. The touch input receiver 25 receives touch operations performed by the user on the touch panel 24. The display 26 is achieved with a liquid crystal display or the like.

The communication interface 27 connects the user terminal 200 to the networks 400 and 500, and communicates with each external device. For example, the communication interface 27 may include short-range communication interfaces such as Bluetooth(R) and Bluetooth Low Energy (BLE), thereby enabling communication with the aerosol-generating device 100.

The image capturer 28 is configured as an inward-facing camera and/or an outward-facing camera in the user terminal 200. In other words, the image capturer 28 has a camera function and stores images and/or videos captured by the user in the storage 53.

The bus 29 interconnects elements and conveys address signals, data signals, and control signals, for example. It is understood by a person skilled in the art that the user terminal 200 may also include a variety of elements other than the above elements. For example, the user terminal 200 may include location information acquisition means for acquiring location information about the user terminal 200 and activity level acquisition means for acquiring the user's activity level (such as a number of steps or a distance traveled, for example).

<1-4. Server>

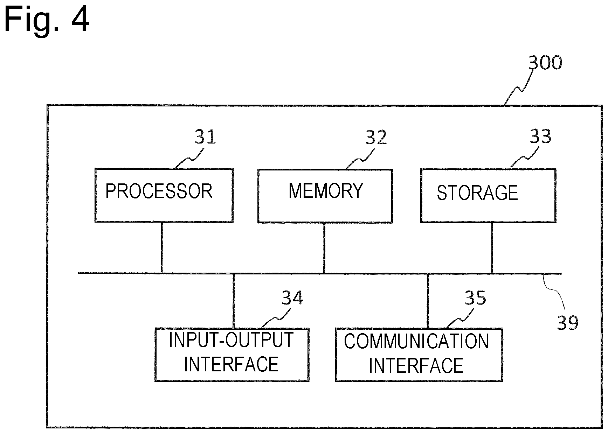

Fig. 4 is a schematic diagram of a hardware configuration of the server 300. The server 300 may be achieved using a general-purpose computer such as a workstation or a personal computer, for example. Alternatively, the server 300 may also be achieved logically by cloud computing.

The server 300 is provided with a processor 31, a memory 32, storage 33, an input-output interface 34, and a communication interface 35, the above elements being electrically connected to each other via a bus 39.

The processor 31, memory 32, storage 33, communication interface 35, and bus 39 are similar to the processor 21, memory 22, storage 23, communication interface 27, and bus 29 in the user terminal 200 illustrated in Fig. 3. In the server 300, the input-output interface 34 receives input from an information input equipment such as a mouse and keyboard, and also provides output to output equipment such as a display.

<2. First embodiments

<2-1. Information processing system>

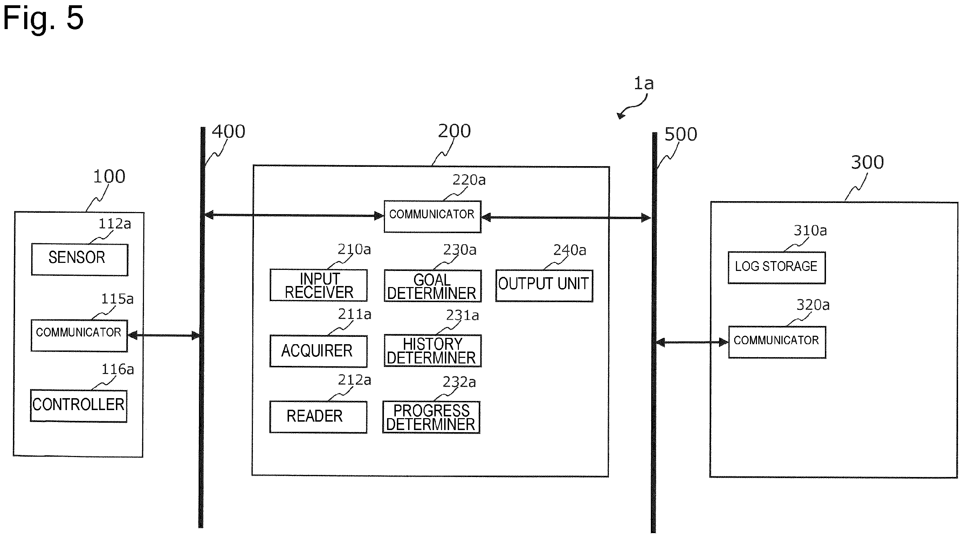

Fig. 5 is a schematic diagram of a functional configuration implemented in an information processing system 1a according to a first embodiment. Specifically, the information processing system 1a for causing a user to transition from the use of the cigarette 150 to the use of the aerosol-generating device 100 is provided with the aerosol-generating device 100 illustrated in Fig. 2, the user terminal 200 illustrated in Fig. 3, and the server 300 illustrated in Fig. 4. In Fig. 5, a major functional configuration implemented in each of the elements is illustrated as blocks. It should be understood by a person skilled in the art that the functional configuration is not limited thereto.

As illustrated in Fig. 2, the aerosol-generating device 100 is provided with a sensor 112a, a communicator 115a, and a controller 116a. The controller 116a causes the sensor 112a to acquire the puff count of the aerosol-generating device 100 and/or the consumption quantity (here, the used number) of the flavor-imparting cartridge 13, for example. The controller 116a also causes the communicator 115a to transmit the information to the user terminal 200.

The user terminal 200 forms an information processing terminal according to the first embodiment. The user terminal 200 is provided with an input receiver 210a, an acquirer 211a, a reader 212a, a communicator 220a, a goal determiner 230a, a history determiner 231a, a progress determiner 232a, and an output unit 240a.

The input receiver 210a receives input from the user, the input including goal information for transitioning from the use of the cigarette 150 to the use of the aerosol-generating device 100 and usage information about the cigarette 150. The input receiver 210a also receives input from the user for editing previously inputted goal information. The acquirer 211a acquires usage information about the aerosol-generating device 100 through communication with the aerosol-generating device 100. The reader 212a is achieved by the image capturer 28 and reads a code (for example, a two-dimensional barcode such as a QR Code(R)) attached to the cigarette 150.

The goal determiner 230a determines a goal value for each predetermined period on the basis of the goal information received by the input receiver 210a. The history determiner 231a determines usage history information for each predetermined period on the basis of inputted usage information about the cigarette 150 received by the input receiver 210a and usage information about the aerosol-generating device 100 acquired by the acquirer 211a. The progress determiner 232a determines progress information with respect to the goal value for each predetermined period on the basis of the goal value determined by the goal determiner 230a and the usage history information determined by the history determiner 231a.

The output unit 240a creates and outputs a progress screen on the basis of the progress information determined by the progress determiner 232a. The output unit 240a also creates and outputs a goal confirmation screen based on the goal value determined by the goal determiner 230a. Furthermore, the output unit 240a respectively creates and outputs a goal setting screen and a history input screen for receiving the input of goal information and usage information about the cigarette 150 through the input receiver 210a. Note that each of the screens created by the output unit 240a will be described later with the use of screen image examples.

The server 300 is provided with log storage 310a and a communicator 320a. The log storage 310a stores various log information processed and acquired by the user terminal 200. The log information includes various information related to the transition from the cigarette 150 to the aerosol-generating device 100. Otherwise, the log information may also include location information and the like acquired by the user terminal 200.

<2-2. Process flow example>

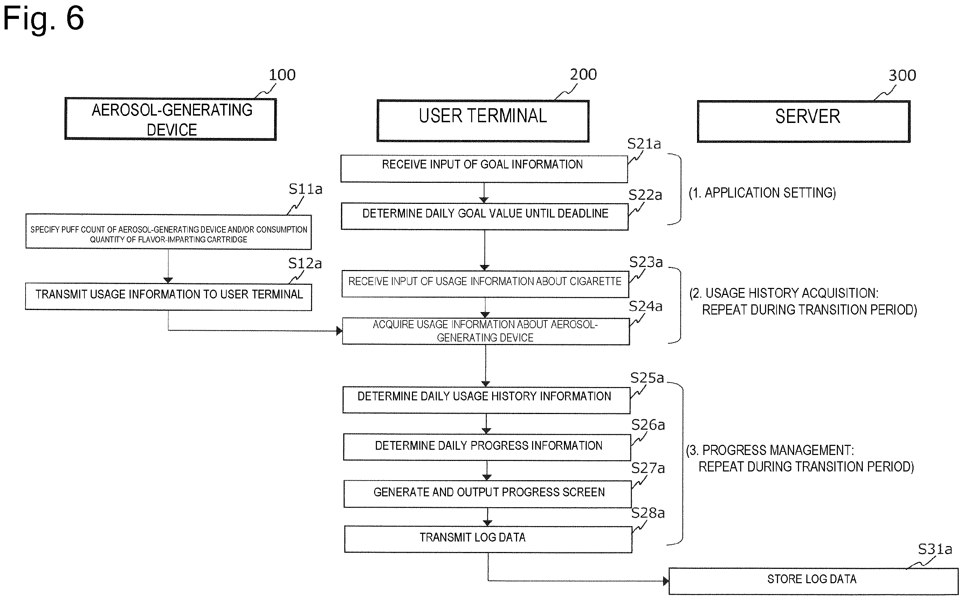

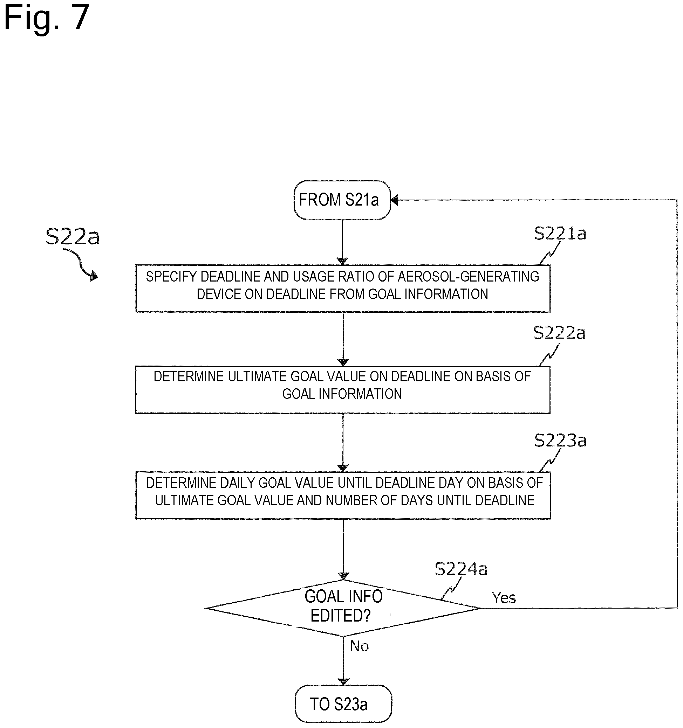

Figs. 6 and 7 will be referenced to describe operations by the information processing system according to the first embodiment. Fig. 6 is a process flowchart of the information processing system according to the first embodiment. Also, Fig. 7 is a detailed flowchart of some of the processing steps in Fig. 6. Through these process flows, a user can be effectively assisted in the transition from the use of the cigarette 150 to the use of the aerosol-generating device 100.

The following illustrates an example of processing steps executed by an information processing device, namely the user terminal 200, according to the present embodiment in cooperation with the aerosol-generating device 100 and the server 300 through communication. Each of the processing steps illustrated herein is merely a non-limiting example, and any other processing steps may also be included. Moreover, the sequence of the processing steps illustrated herein is merely a non-limiting example, and in some cases, the processing steps may also be executed in a different sequence.

The series of processing steps by the user terminal 200 can be divided into the three stages of (1) an application setting stage, (2) a usage history acquisition stage, and (3) a progress management stage. The application setting stage is performed when setting up the application 250. Note that the application setting stage may be performed not only during initial setting, but also in a reconfiguration during a transition period. On the other hand, the usage history acquisition stage and the progress management stage are performed repeatedly during the transition period.

<2-2-1 . Application setting stage>

First, in step S21a, the input receiver 210a receives, through the goal setting screen, user input of goal information for transitioning from the use of the cigarette 150 to the use of the aerosol-generating device 100. The inputted goal information includes a deadline for the transition (or a period for the transition).

Next, in step S22a, the goal determiner 230a determines a goal value for each predetermined period until the deadline on the basis of the inputted goal information. Here, the predetermined period is assumed to be set to one day.

More specifically, as illustrated in Fig. 7, step S22a includes steps S221a to S224a executed by the goal determiner 230a. First, from the goal information inputted in step S21a, the day of the deadline (that is, the last day) and a usage ratio of the aerosol-generating device 100 on the deadline day are specified (step S221a). Next, an ultimate goal value for the deadline day is determined (step S222a), and in addition, a daily goal value until the deadline day is specified (step S223a).

The ultimate goal value includes a usage goal value (for example, a planned number to be used) for the cigarette 150 on the deadline day and/or a usage goal value (for example, a target number of the flavor-imparting cartridges 13 to be used and/or a target number of puffs to be taken on the aerosol-generating device 100) for the aerosol-generating device 100 on the deadline day. Also, the daily goal value includes a daily planned usage value (for example, a planned number to be used) for the cigarette 150 and/or a daily planned usage value (for example, a planned number of the flavor-imparting cartridges 13 to be used and/or a number of puffs to be taken on the aerosol-generating device 100) for the aerosol-generating device 100. Such a daily planned usage value for the cigarette 150 and/or daily planned usage value for the aerosol-generating device 100 may be calculated on the basis of the ultimate goal value and the number of days until the deadline day.

When the daily goal value is determined in step S22a, thereafter, the output unit 240a can generate and output a daily goal confirmation screen indicating the goal value until the deadline day.

In this way, steps S21a and S22a described above are executed when the user who desires to transition from the use of the cigarette 150 to the use of the aerosol-generating device 100 sets up the application 250 on the user terminal 200. Accordingly, a transition plan until the desired deadline that is customized for each user can be determined.

Note that the user can further edit previously inputted goal information. That is, the input receiver 210a may additionally execute a step for receiving input for editing the goal information (step S224a in Fig. 7). Once the goal information is edited, the goal determiner 230a recalculates the goal value for previously determined future dates on the basis of the edited goal information (steps S221a to S223a). Specifically, the goal value from the time of editing until the deadline is recalculated.

In this way, the user can flexibly reconfigure goals in accordance with the user's situation, even in the middle of the transition period after the goal information is initially set. This arrangement is user-friendly as it allows the user to receive flexible transition assistance.

<2-2-2. Usage history acquisition stage>

When the application setting stage is performed and a transition plan including a goal value until the desired deadline is determined, in the usage history acquisition stage, the user terminal 200 repeatedly obtains usage information about the cigarette 150 and usage information about the aerosol-generating device 100 during the transition period. The information obtained at this point is used by the user terminal 200 to manage the status of the user's transition.

In step S23a, the input receiver 210a receives user input of usage information about the cigarette 150. Specifically, every time the user uses the cigarette 150 (that is, every time the user smokes a single cigarette 150), the input receiver 210a receives the input of usage information about the cigarette 150 through a prescribed user interface image (described later) provided on the history input screen. The usage information about the cigarette 150 is associated with the time at which the input is received.

Note that the usage information about the cigarette 150 includes a brand selected from among a plurality of brands applicable to the cigarette 150. Information pertaining to the plurality of brands is preregistered. Specifically, a code (for example, a two-dimensional barcode) is attached to a package containing the cigarette 150, and information can be registered in the user terminal 200 by having the reader 212a read the code. Thereafter, if the user causes the reader 212a to read the code on the package, the brand to be selected may be specified automatically. With this arrangement, the user terminal 200 can obtain usage information regarding the number of the cigarettes 150 smoked by the user efficiently in association with the corresponding brand.

On the other hand, in step S24a, the acquirer 211a acquires usage information about the aerosol-generating device 100 directly from the aerosol-generating device 100 through communication. On the aerosol-generating device 100 side, in step S11a, every time the user uses the aerosol-generating device 100, the sensor 112a detects the numbers of puffs taken on the aerosol-generating device 100 and/or the consumption quantity (here, the used number) of the flavor-imparting cartridge 13. In other words, usage information about the aerosol-generating device 100 is specified internally in association with the usage time.

Thereafter, every time communication is established and interaction occurs between the aerosol-generating device 100 and the user terminal 200, in step S12a, the communicator 115a of the aerosol-generating device 100 transmits usage information about the aerosol-generating device 100 to the user terminal 200. At this point, it is sufficient to transmit only the difference from the most recently transmitted usage information.

In this way, in the usage history acquisition stage, the user terminal 200 can obtain in detail both usage information about the cigarette 150 and usage information about the aerosol-generating device 100 during the transition period. The usage information is treated as data to be processed and managed in the following progress management stage.

<2-2-3. Progress management stage>

If the usage history acquisition stage is performed during the transition period and usage information about the cigarette 150 and usage information about the aerosol-generating device 100 is obtained, the user terminal 200 presents progress information to the user as appropriate by performing data processing and visualization using the usage information.

First, in step S25a, the history determiner 231a determines actual daily usage history information on the basis of the usage information about the cigarette 150 and the usage information about the aerosol-generating device 100. As described above, the time of reception by the input receiver 210a is associated with the usage information about the cigarette 150, and the usage time is associated with the usage information about the aerosol-generating device 100. In other words, by performing aggregation on the basis of the usage times, the daily usage history information can be determined. That is, the usage history information includes the daily used number of the cigarettes 150 and the daily used number of the flavor-imparting cartridges 13 of the aerosol-generating device 100 and/or the daily puff count of the aerosol-generating device 100.

Next, in step S26a, the progress determiner 232a determines daily progress information with respect to the goal value on the basis of the goal value determined in step S22a and the usage history information determined in step S25a.

The progress information may include a daily score. Specifically, the progress determiner 232a determines the daily usage ratio of the aerosol-generating device 100 on the basis of the daily used number of the cigarettes 150 and the daily used number of the flavor-imparting cartridges 13 of the aerosol-generating device 100 and/or the daily puff count of the aerosol-generating device 100. Thereafter, the daily score is calculated on the basis of the ratio of the daily planned usage value of the aerosol-generating device 100 determined in step S22a and the daily usage ratio of the aerosol-generating device 100. The score may be treated as an indicator that indicates a daily achievement percentage.

The progress information may also include a daily achievement result ranked on the basis of the daily score. Specifically, a rank is associated with a predetermined range of values, and the progress determiner 232a determines the daily achievement result by determining which rank (for example, an evaluation of ○/△/×) the calculated score corresponds to. Additionally, a message corresponding to the pattern of progress may be pre-associated with the progress information including such a rank.

Furthermore, the progress information may include the value of a prescribed indicator calculated on the basis of the usage information about the cigarette 150 and/or the usage information about the aerosol-generating device 100.

In step S27a, in response to a user request, the output unit 240a creates and outputs a progress screen on the basis of the progress information determined in step S26a. The progress screen may include at least one of a display of the score, a display of a calendar with visually associated achievement results, or a display of a graph illustrating how the value of the prescribed indicator changes daily over time.

At the end of the progress management stage, in step S28a, the communicator 220a transmits various data obtained by executing steps S25a to S27a to the server 300 as log data. The log data may include various data such as location information about the user terminal 200 and the like.

In response, in step S31a, the communicator 320a of the server 300 receives the log data and the log storage 310a stores the log data. The processes (steps S25a to S28a and step S31a) of the progress management stage are continued repeatedly during the transition period until the deadline day is reached.

In this way, in the progress management stage, the user terminal 200 determines daily progress information during the transition period, and creates and outputs a detailed progress screen. With this arrangement, the user is able to manage progress effectively with respect to the user's own transition.

<2-3. Display screen implementation examples>

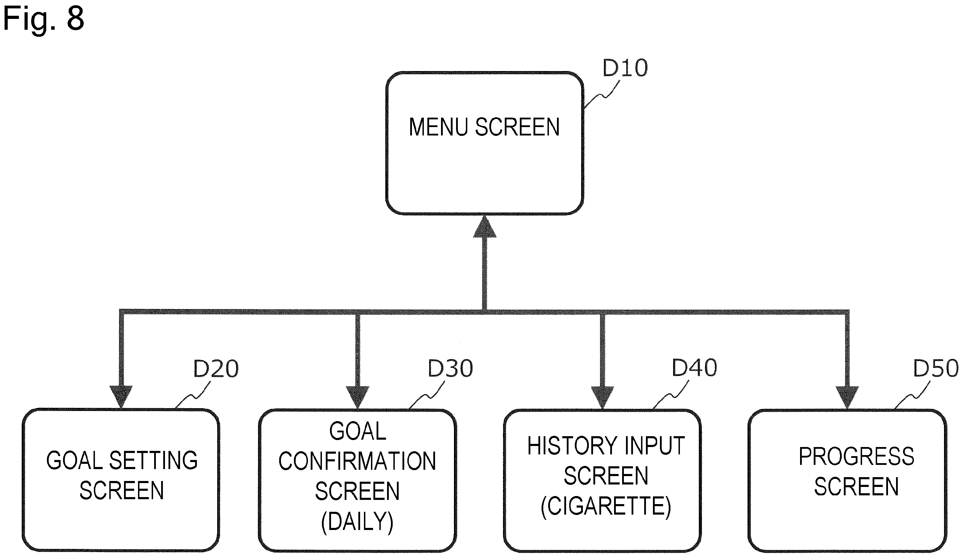

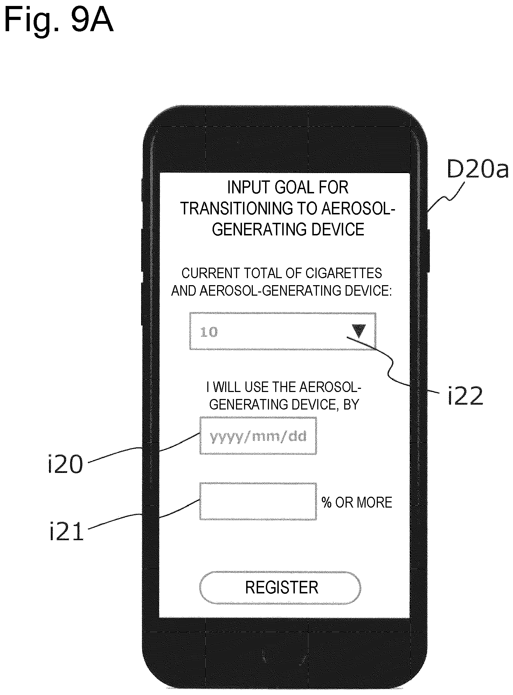

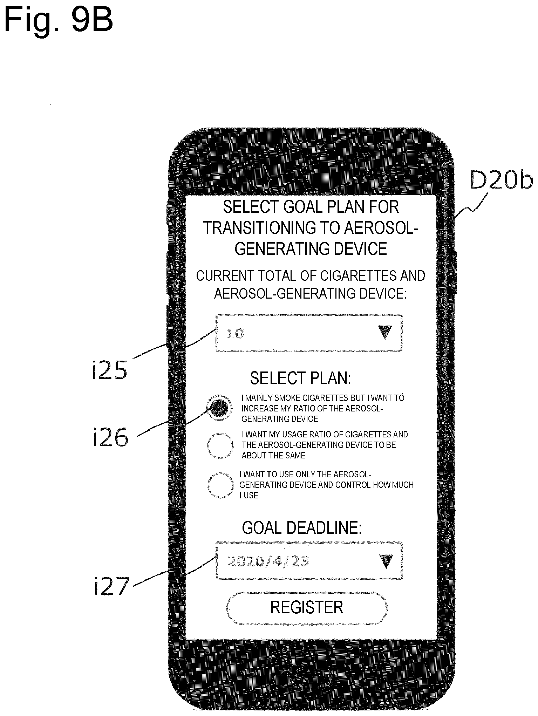

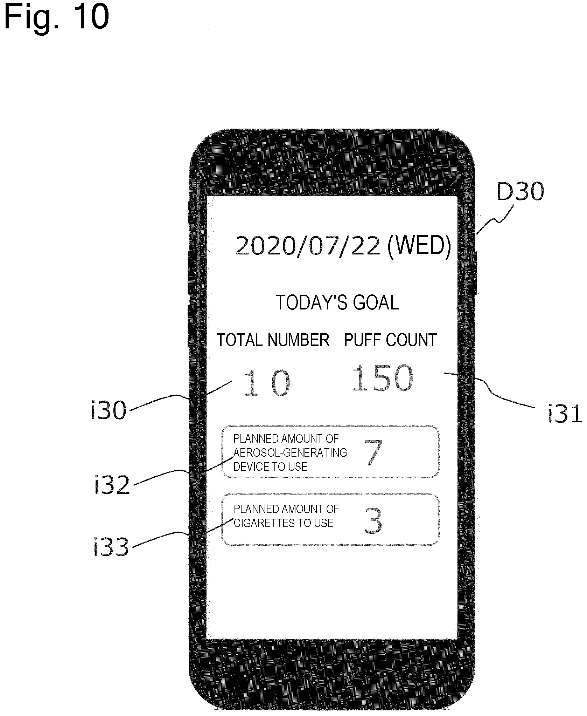

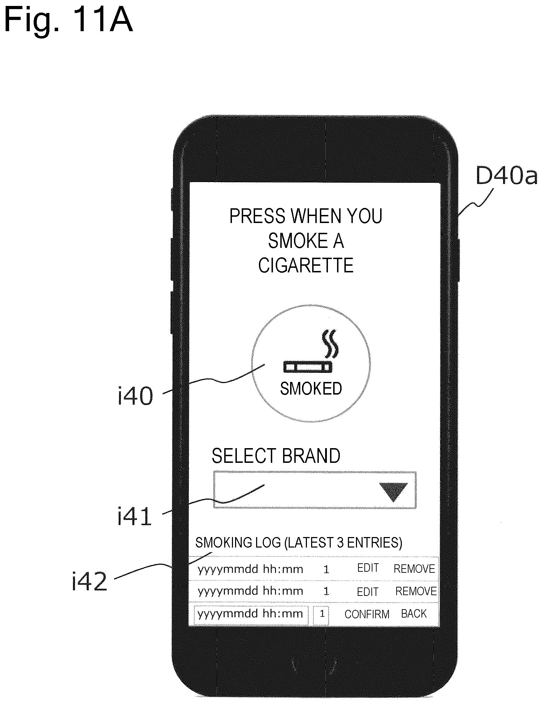

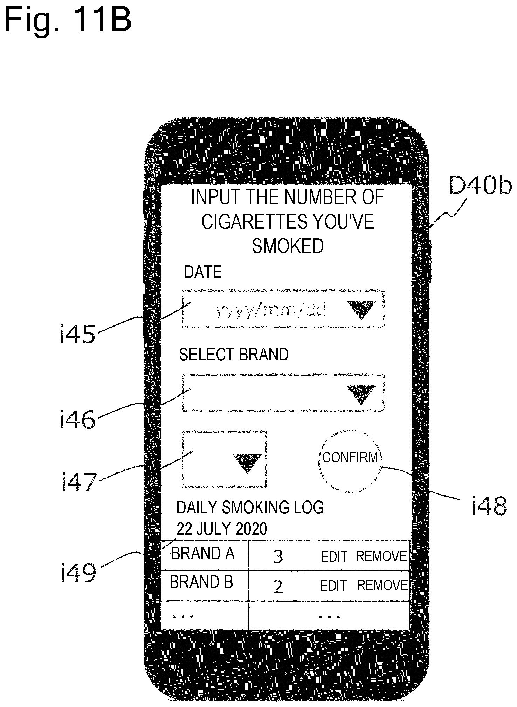

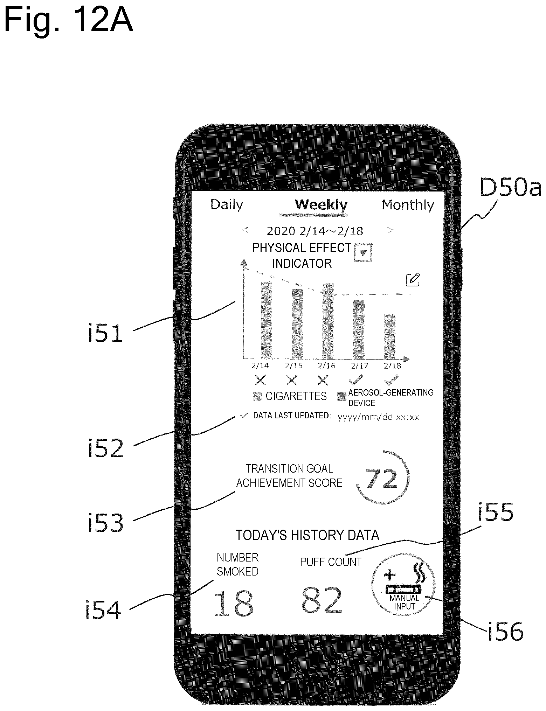

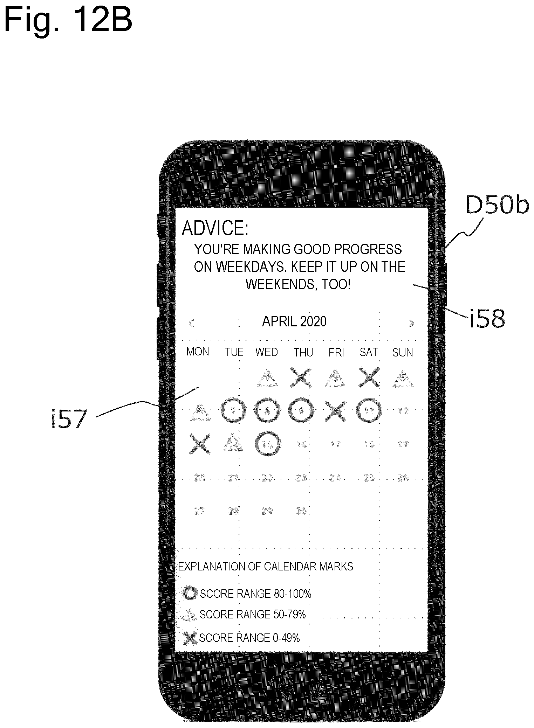

Figs. 8 to 12B will be referenced to describe implementation examples of each screen image displayed on the user terminal 200 through the process flow illustrated in Fig. 6, along with specific process examples. Fig. 8 is a schematic transition diagram of each screen image. Figs. 9A and 9B are examples of goal setting screen images. Fig. 10 is an example of a goal confirmation screen image. Figs. 11A and 11B are examples of history input screen images. Figs. 12A and 12B are examples of progress screen images.

As illustrated in Fig. 8, the user can switch between screen images through the application 250 on the user terminal 200. Each screen image is created and displayed by the output unit 240a. The screens to be displayed include a menu screen D10, a goal setting screen D20, a daily goal confirmation screen D30, a cigarette history input screen D40, and a progress screen D50. It may be possible to switch from one screen to another. Note that the screens and screen changes displayed herein and the items displayed on each screen are merely examples, and it is understood by a person skilled in the art that the configuration is not necessarily limited to these examples.

When the user launches the application 250, the menu screen D10 is displayed first. The user can switch from the menu screen D10 to a desired screen (D20 to D50). The goal setting screen D20 is a screen for receiving the input of goal information in step S21a. The goal confirmation screen D30 is a screen indicating the daily goal value until the transition deadline day as a result of step S22a. The history input screen D40 is a screen for receiving the input of usage information about the cigarette 150 in step S23a. The progress screen D50 is a screen created in step S27a on the basis of the progress information determined in step S26a.

<2-3-1. Goal setting screen implementation example>

On a goal setting screen D20a in one example illustrated in Fig. 9A, specific goal information for transitioning from the cigarette to the aerosol-generating device is "inputted" by the user. Otherwise, on a goal setting screen D20b in another example illustrated in Fig. 9B, goal information such as a candidate plan regarding the transition is "selected" by the user. The user can set a transition goal by using a desired screen in either Fig. 9A or 9B. Note that it is also possible to implement only one screen in either Fig. 9A or Fig. 9B as the goal setting screen.

Specifically, on the goal setting screen D20a in Fig. 9A, the goal information to be inputted by the user includes a deadline day (i20) for the transition, a planned usage ratio (i21) of the aerosol-generating device on the deadline day, and a current total used number (i22) for the cigarette and the aerosol-generating device.

More specifically, for the deadline day (i20), the user may use an input field here to input any date in yyyy/mm/dd format. Alternatively, the user may input any period. Also, the planned usage ratio (i21) may be the ratio of the planned number of the flavor-imparting cartridges to be used (that is, the usage ratio of the aerosol-generating device) with respect to the combined total of the planned number of the cigarettes to be used and the planned number of the flavor-imparting cartridges of the aerosol-generating device to be used on the deadline day. The user may use an input field to input such a planned usage ratio (i21) directly (such as a usage ratio of "30%" for the aerosol-generating device, for example). Alternatively, the user may directly input a relative reduction ratio (such as a reduction ratio of "70%", for example) of the planned number of the cigarettes to be used on the deadline day. Note that with regard to the planned usage ratio (i21), instead of having the user input a numerical value directly into an input field, a value in the range from 0% to 100% in 10% intervals may be displayed and selectable from a pull-down menu accessed through the selection field.

Also, the current total used number (i22) may be determined by the user and set to any number selected from a pull-down menu. Note that the user may also select a current consumed number for the cigarette and a current consumed number for the aerosol-generating device separately.

Otherwise, on the goal setting screen D20b in Fig. 9B, the goal information to be selected by the user includes the current total used number (i25) of the cigarette and the aerosol-generating device, a plan (i26) selected from among a plurality of plans, and a goal deadline day (i27).

More specifically, the current total used number (i25) is similar to the current total used number (i22) in Fig. 9A. In other words, the current total used number (i25) may be determined by the user and set to any number selected from a pull-down menu. Note that the user may also select a current consumed number for the cigarette and a current consumed number for the aerosol-generating device separately.

The plan (i26) may be selected by having the user use a radio button to select one plan from among a plurality of predetermined plans. Examples of plans may be, but are not limited to, "I mainly smoke cigarettes but I want to increase my ratio of the aerosol-generating device", "I want my usage ratio of cigarettes and the aerosol-generating device to be about the same", "I want to use only the aerosol-generating device", "I want to use only the aerosol-generating device and control how much I use", and so on. A planned consumption ratio (corresponding to i21 above) for determining the ultimate goal value is pre-associated with each plan according to a setting. Note that plans suited to the user may also be selectively displayed and recommended on the basis of user attribute information, lifestyle information surveyed in advance, and the like registered during the initial setting of the application.

Also, for the goal deadline day (i27), any date may be displayed and selected by the user from a pull-down menu.

Note that in the example in Fig. 9B, "10" is selected as the current total used number (i25), "I mainly smoke cigarettes but I want to increase my ratio of the aerosol-generating device" is selected as the plan (i26), and "2020/04/23" is selected as the deadline day (i27). In this case, the "I mainly smoke cigarettes but I want to increase my ratio of the aerosol-generating device" plan is assumed to contain a preset value so that planned consumption ratio of the flavor-imparting cartridge on the deadline day is "30%" (that is, the ratio of the planned number of the cigarettes to be consumed is "70%").

As a result, in step S221a of Fig. 7 described above, the goal determiner 230a specifies "2020/04/23" as the deadline day and "30%" as the planned usage ratio of the aerosol-generating device on the deadline day. In the subsequent step S222a, the goal determiner 230a additionally sets the usage goal value (here, the goal number of the flavor-imparting cartridges 13 to be used) of the aerosol-generating device to "3" (10 × 30%) as the ultimate goal value on the deadline day. In this case, the usage goal value (here, the planned number to be used) for the cigarette is also set to "7" (10 × (10 - 3) × 10%) as the ultimate goal value on the deadline day.

In another example, "10" is selected as the current total used number (i25), "I want to use only the aerosol-generating device and control how much I use" is selected as the plan (i26), and "2020/04/23" is selected as the deadline day (i27). Note that the "I want to use only the aerosol-generating device and control how much I use" plan is assumed to contain a preset value so that the reduction ratio of the total used number on the deadline day is "40%" with respect to the day the goal is set.

As a result, in step S221a of Fig. 7 described above, the goal determiner 230a specifies "2020/04/23" as the deadline day and "100%" as the usage ratio of the aerosol-generating device on the deadline day. Also, in the subsequent step S222a, the goal determiner 230a additionally sets the usage goal value (here, the goal number of the flavor-imparting cartridges 13 to be used) of the aerosol-generating device to "6" (10 × (10 - 4) × 10%) as the ultimate goal value on the deadline day. In this case, the usage goal value (here, the planned number to be used) for the cigarette is also set to "0" (10 × 0 × 10%) as the ultimate goal value on the deadline day.

Note that, as described above, the user can further edit previously inputted goal information (Fig. 7: step S224a). That is, the user can edit the goal information by re-inputting information for each input item on the goal setting screen D20. In this case, the goal determiner 230a recalculates the goal value from the current day until the deadline day. Note that the progress information, that is, the history already calculated up to the editing day, does not have to be recalculated.

<2-3-2. Goal confirmation screen implementation example>

In step S223 in Fig. 7, the goal determiner 230a additionally determines the daily goal value until the deadline day with respect to the usage goal value (that is, the ultimate goal value) on the deadline day determined above. Thereafter, the determined daily goal value is displayed on the goal confirmation screen D30.

The daily goal value is displayed on the goal confirmation screen D30 in one example illustrated in Fig. 10. The goal value includes a total planned number to be used for the day (i30) during the transition period, a total puff count for the day (i31), a daily goal value which is the planned usage value (here, the planned number of the flavor-imparting cartridges 13 to be used) of the aerosol-generating device (i32), and a daily goal value which is the planned usage value (here, the planned number to be used) for the cigarette (i33). That is, the value of each item corresponding to the day designated by the user is displayed on the goal confirmation screen D30. More specifically, the example in Fig. 10 is for the date "2020/07/22 (Wed)", but data up to the goal deadline day (i27) is displayable for each day.

In the example in Fig. 10, "10" is displayed as the total planned number to be used for the day (i30), to which the value inputted as the current total used number (i25) in Fig. 9B is directly applied. Also, "150" is the total puff count for the day (i31), to which the value corresponding to the current total used number (i25) is directly applied. Here, the total puff count for the day is calculated as 15 puffs per product. In the example in Fig. 9B, "10" is multiplied by "15 puffs/product" to calculate "150" (10 × 15). The number of puffs per product is not limited to 15 and may be a prescribed number such as 18 or 20. Note that the value of each of the total planned number to be used (i30) and the total puff count (i31) do not have to be changed throughout the transition period.

In the example in Fig. 10, "7" is displayed as the planned usage value (i32) of the aerosol-generating device and "3" is displayed as the planned usage value (i33) for the cigarette. These values may be calculated as follows. Note that the following calculation example assumes that the current total number to be used (i25) is "10", of which "3" is the current used number for the aerosol-generating device and "7" is the current used number for the cigarette. Also, the usage goal value (that is, the ultimate goal value) of the aerosol-generating device on the deadline day is assumed to be "7" and the usage goal value of the cigarette is assumed to be "3". Furthermore, the number of days (remaining days) until the deadline day is assumed to be 120 days.

In this case, for the cigarette, the daily goal value is gradually decreased so that the used number will decrease by 4 (-4) after 120 days to meet the deadline. Meanwhile, for the aerosol-generating device, the daily goal value is gradually increased so that the used number will increase by 4 (+4) after 120 days to meet the deadline.

That is, the decrease in the daily planned usage value for the cigarette may be set to -4 120 days = -0.0333 per day, and the increase in the daily planned usage value of the aerosol-generating device may be set to +4 120 days = +0.0333 per day. However, the numbers of the products need to be integer values. That is, the values to be actually displayed may be set to integer values rounded up by raising the fractional part using a ceiling function. It is understood by a person skilled in the art that, in this example, the planned usage value (i33) for the cigarette decreases by 1 and the planned usage value (i32) for the aerosol-generating device increases by 1 every 30 days.

When formulated, the above example can be expressed as follows. The value of the planned usage value (i32) of the aerosol-generating device is calculated as

value of i32 = ceiling (current used number + (((usage goal value - current used number) ÷ (remaining days until deadline)) × (remaining days until deadline))) ... (Expression 1)

. Note that the current used number and the usage goal value are both values related to the aerosol-generating device.

Also, the function ceiling(x) represents a ceiling function. Note that a ceiling function maps a real value x to the smallest integer value equal to or greater than x.

On the other hand, the value of the planned usage value (i33) for the cigarette is calculated as

$$\text{value of i33} = \text{ceiling (current used number} - (((\text{current used number} - \text{usage goal value}) \div (\text{remaining days until deadline})) \times$$
$$(\text{remaining days until deadline}))) \ldots (\text{Expression 2}).$$

Note that the current used number and the usage goal value are both values related to the cigarette.

Instead of using both Expression 1 and Expression 2 above, either Expression 1 or Expression 2 may be used to calculate either the value of i32 or the value of i33, and the value of the other may be calculated by subtracting the calculated value from the current total used number (i25).

<2-3-3. Goal setting screen implementation example>

Through the history input screen D40a for the cigarette in one example illustrated in Fig. 11A, the user inputs usage information every time the user smokes a cigarette. Note that to keep the user from forgetting to input information, the user terminal 200 may execute a push notification (reminder) at any given time, such as every morning at 8:00, and any number of times to encourage user input.

The usage information about the cigarette inputted by the user on the goal setting screen D40a in Fig. 11A includes brand information (i41) and the most recent smoking log (i42). Additionally, a circular user interface image (i40) indicating a smoking mark is displayed along with text information stating "Smoked" on the history input screen D40a. If the user touches the user interface image (i40), a smoking log is registered together with the current time and displayed as the most recent smoking log (i42). In the case of registering a smoking log including multiple smoking products, the user may simply touch the user interface image (i40) consecutively or maintain (that is, hold) the touch state. At this time, one type of brand information (i41) selected via a selection field from among a plurality of pre-registered brands may be associated.

As described above, a code (for example, a two-dimensional barcode) is attached to a package containing the cigarette, and information is registered in the user terminal by having the user terminal read the code. Thereafter, when the cigarette is used, the user may cause the code on the package to be read and thereby cause the brand that should be selected to be specified automatically. This arrangement can reduce the user burden. Obviously, the selection of one type of brand information (i41) may also be performed through a manual operation by the user.

In the smoking log (i42), multiple entries may be displayed in order from newest to oldest. Also, to handle incorrect inputs by the user, the user can correct the smoked number by "editing". The user can also "remove" the most recent smoking log itself. Data may not be updated in response to these editing and removal operations unless the user touches the word "Confirm".

Instead of the history input screen D40a for the cigarette in one example illustrated in Fig. 11A, a history input screen D40b for the cigarette in another example illustrated in Fig. 11B may be applied. On the history input screen D40b, the usage information about the cigarette to be registered by the user includes a smoking date (i45), brand information (i46), a smoked number (i47), and a daily smoking log (i49) selected via selection fields. Also, on the history input screen D40b, a circular user interface image (i48) indicating "Confirm" is displayed.

The user performs selection operations with respect to the smoking date (i45), the brand information (i46), and the smoked number (i47) displayed in a pull-down menu, and then touches the user interface image (i48). With this arrangement, a smoking log is registered and reflected in the daily smoking log (i49).

Also, on the history input screen D40b, the daily smoking log (i49) may be displayed in association with the smoked number for each brand. For instance, in the example in Fig. 11B, an indication that the user has smoked "3" of "Brand A" is displayed. Also, in the daily smoking log (i49), the smoked number can be corrected by "editing" and the log itself can be "removed", similarly to the smoking log (i42) in Fig. 11A.

Note that the way in which a push notification (reminder) is sent at any given time and any number of times and the preferable way in which brand information is registered and selected by reading a code attached to the package of the cigarette are similar to what is described on the history input screen D40a.

<2-3-4. Progress screen implementation example>

On a progress screen D50a in one example illustrated in Fig. 12A, daily progress information about the transition from the cigarette to the aerosol-generating device is outputted. Progress information displayed on the progress screen D50a includes: a graph (i51) of a predetermined period in which the value of a prescribed indicator (for example, a physical effect indicator) changes daily over time; the last data update date and time (i52); a transition goal achievement score (i53); and the total of the consumption quantity (herein, the used number) of the aerosol-generating device and the used number of the cigarettes (i54) and the total puff count (i55) acquired from the aerosol-generating device which are included in the daily usage history information for the day.

The graph (i51) of the predetermined period is user-selectable from among daily, weekly, and monthly periods, and the selected period is set to the horizontal axis of the graph. On the vertical axis of the graph, the used number of tobacco products and/or the value of the prescribed indicator are set. The graph may be displayed as a cumulative bar graph in which totals of the used number for the aerosol-generating device and the used number for the cigarette are each color-coded, with the values of the prescribed indicator overlaid as a line graph (dashed line). Note that a graph going back a predetermined period into the past from the present may also be displayed.

Here, the values of the prescribed indicator may include a value of a physical effect indicator, a quantity of nicotine, and a tar value. These values are calculated on the basis of the value of the used number for the aerosol-generating device and the value of the used number for the cigarette. For example, the value of the physical effect indicator may be calculated according to the formula

$$\text{value of physical effect indicator} = \text{used number of aerosol-generating device} \times 0.1 + \text{used number of cigarettes} \times 1.0$$

but is not limited thereto. Note that the set values "0.1" and "1.0" in the above formula can be changed successively. Also, an achievement result described later (herein, a mark such as ✕) may be displayed below dates on the horizontal axis.

The last data update date and time (i52) expresses the date and time of the last time when communication was established and interaction occurred between the aerosol-generating device 100 and the user terminal 200. Note that in response to the establishment of communication between the two, the last data update date and time (i52) is updated and the acquisition of various data from the aerosol-generating device 100 is executed. By checking the display of the last data update date and time (i52), the user can determine whether the data being displayed on the progress screen D50a is up to date. Note that if the data is up to date, an indication thereof (herein, a check mark) may be displayed.

The transition goal achievement score (i53) indicates an average score over the user-selected predetermined period (herein, in units of weeks) by calculating the daily scores. In one non-limiting example, the daily scores may be a transition achievement percentage (maximum of 100%) calculated as the percentage of the ratio of the daily planned usage value of the aerosol-generating device with respect to the actual daily usage ratio of the aerosol-generating device.

For example, the ratio of the daily planned usage value of the aerosol-generating device may be calculated according to the formula

$$\text{ratio of daily planned usage value of aerosol-generating device} = \text{planned usage value of aerosol-generating device for the day} / (\text{planned usage value of cigarettes for the day} + \text{planned usage value of aerosol-generating device for the day}) \times 100$$

but is not limited thereto.

Also, the actual daily usage ratio of the aerosol-generating device may be calculated according to the formula

$$\text{actual daily usage ratio of aerosol-generating device} = \text{usage information about aerosol-generating device for the day (actual value)} / (\text{usage information about cigarette for the day (actual value)} + \text{usage information about aerosol-generating device for the day (actual value)}) \times 100$$

but is not limited thereto.

The total of the used number for the aerosol-generating device and the used number for the cigarette may be aggregated as the (total) tobacco product (i54) for the day, the total of the puff count for the day acquired from the aerosol-generating device may be aggregated as the (total) puff count (i55), and the results may be displayed on the progress screen D50a.

A circular user interface image (i56) indicating a smoking mark is also displayed on the progress screen D50a. If the user touches the user interface image (i56), the display may switch to the history input screen D40 described above. With this arrangement, the registration of the history by the user and the display of the results thereof can be linked more dynamically.

Otherwise, a progress screen D50b in another example illustrated in Fig. 12B includes a display of a calendar with visually associated achievement results. That is, the progress information in this case includes a daily achievement result (i57) ranked on the basis of the daily score (achievement percentage) and a comment (i58) related to the achievement percentage.

Specifically, in the example of the progress screen D50b illustrated in Fig. 12B, a standard is preset such that a score in the range from 0% to 49% is "×", a score in the range from 50% to 79% is "△", and a score in the range from 80% to 100% is "O", and the daily achievement result (i57) is ranked accordingly. Such achievement results may be overlaid onto the calendar displayed on the progress screen D50b. With this arrangement, the user can see the status of their own achievement at a glance.

Also, prepared comments related to the achievement percentage is associated with the pattern of change over time in the achievement percentage. In addition, one of the comments (i58) related to the achievement percentage may be associated with the pattern of the user for the day and displayed on the progress screen D50b. In the example of the progress screen D50b illustrated in Fig. 12, the advice "You're making good progress on weekdays. Keep it up on the weekends, too!" is associated and displayed in the upper part of the screen. In this example, the achievement percentage results were good in the week from 6-12 April 2020, and a message pre-associated with the pattern is displayed.

In this way, according to the present embodiment, the user is effectively encouraged to transition from the use of the cigarette to the use of the aerosol-generating device.

<3. Information processing system according to second embodiments

<3-1. Information processing system>

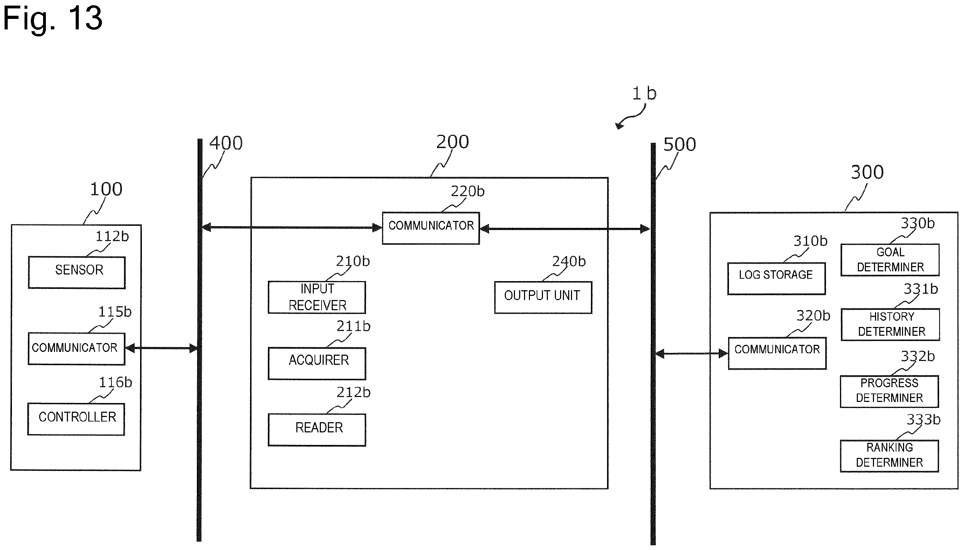

Fig. 13 is a schematic diagram of a functional configuration implemented in an information processing system 1b according to a second embodiment. Specifically, the information processing system 1b for causing a user to transition from the use of the cigarette 150 to the use of the aerosol-generating device 100 is provided with the aerosol-generating device 100, the user terminal 200, and the server 300, similarly to the information processing system 1a according to the first embodiment.

A sensor 112b, a communicator 115b, and a controller 116b of the aerosol-generating device 100 are similar to the sensor 112a, the communicator 115a, and the controller 116a of the aerosol-generating device 100 according to the first embodiment.

The user terminal 200 is provided with an input receiver 210b, an acquirer 211b, a reader 212b, a communicator 220b, and an output unit 240b. These elements are similar to the input receiver 210a, the acquirer 211a, the reader 212a, the communicator 220a, and the output unit 240a of the user terminal 200 according to the first embodiment.

The server 300 includes log storage 310b, a communicator 320b, a goal determiner 330b, a history determiner 331b, a progress determiner 332b, and a ranking determiner 333b. The log storage 310b and the communicator 320b are similar to the log storage 310a and the communicator 320a of the server 300 according to the first embodiment. Also, the goal determiner 330b, the history determiner 331b, and the progress determiner 332b have functions similar to the goal determiner 230a, the history determiner 231a, and the progress determiner 232a provided in the user terminal 200 in the first embodiment.

The ranking determiner 333b of the server 300 determines a ranking (described later) of a plurality of registered users on the basis of user progress information determined by the progress determiner 332b.

<3-2. Process flow example>

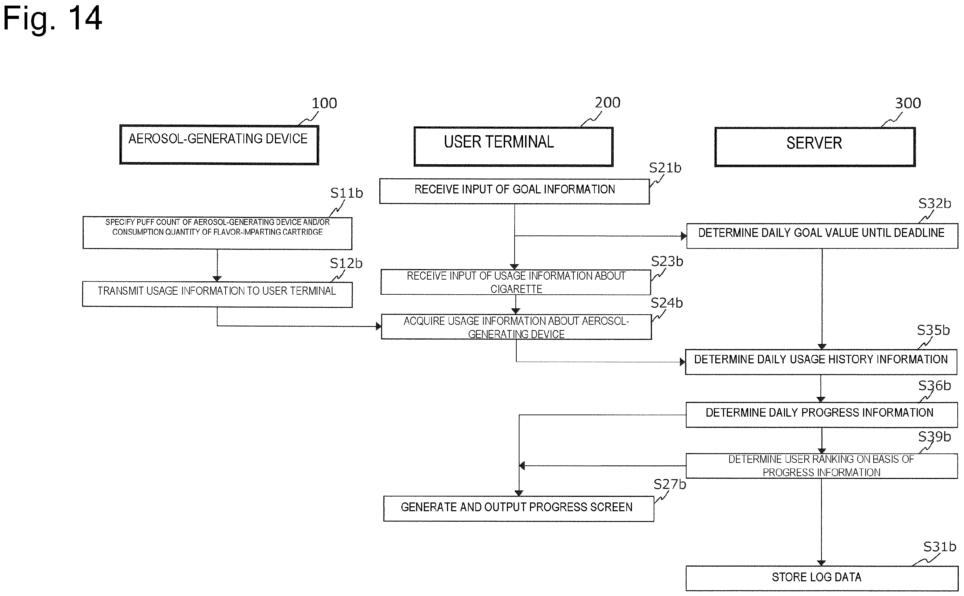

Fig. 14 is a process flowchart of the information processing system 1b according to the second embodiment. Through this process flow, a user can be effectively assisted in the transition from the use of the cigarette 150 to the use of the aerosol-generating device 100.

Fig. 14 illustrates an example of processing steps executed by the user terminal 200 in cooperation with the aerosol-generating device 100 and the server 300 through communication. Each of the processing steps illustrated herein is merely a non-limiting example, and any other processing steps may also be included. Moreover, the sequence of the processing steps illustrated herein is merely a non-limiting example, and in some cases, the processing steps may also be executed in a different sequence.

Steps S11b and S12b executed by the aerosol-generating device 100 are similar to steps S11a and S 12a executed by the aerosol-generating device 100 in the first embodiment.

Also, steps S21b, S23b, S24b, and 27b executed by the user terminal 200 are similar to steps S21a, S23a, S24a, and 27a executed by the user terminal 200 in the first embodiment.

Furthermore, step S31b (that is, the step for storing log data) executed by the server 300 is similar to step S31a executed by the server 300 in the first embodiment.

Additionally, steps S32b, S35b, and S36b executed by the server 300 are similar to steps S22a, S25a, and S26a executed by the user terminal 200 in the first embodiment. That is, in the present embodiment, the entity that executes the above processes is the server 300 instead of the user terminal 200 in the first embodiment. Consequently, in the present embodiment, the exchange of data is executed as appropriate between the communicator 220b of the user terminal 200 and the communicator 320b of the server 300.

Next, in step S39b executed by the server 300, the ranking determiner 333b determines a user ranking on the basis of the user progress information determined by the progress determiner 332b. For example, the daily scores for a certain user averaged over one week may be used to determine the ranking of the user among all users. Note that the determined user ranking may be displayed on the progress screen displayed by the output unit 240b of the user terminal 200.

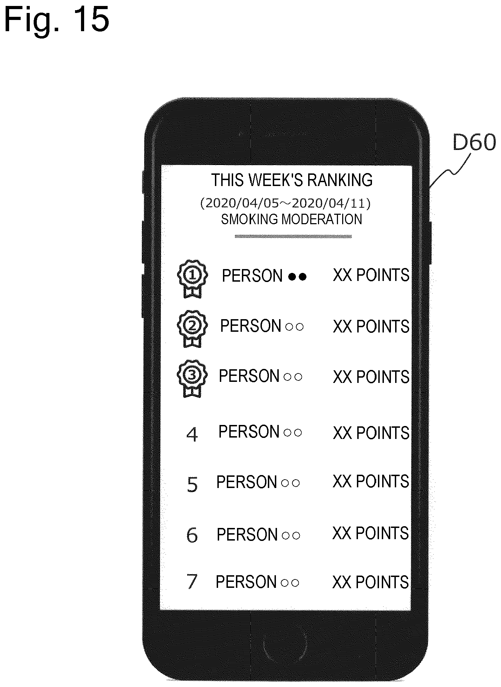

Fig. 15 is an example image of a ranking screen D60 displayed on the user terminal 200 in step S39b. In this case, the smoking moderation statuses (that is, the weekly average scores) of users for the week from 5 April 2020 to 11 April 2020 are ranked.

<4. Other embodiments>

(1) In the first or second embodiment, the user terminal 200 determines the daily used number of the cigarettes 150 included in the usage history information on the basis of the usage information about the cigarette 150 inputted by the user. In contrast, in another embodiment, the user terminal 200 may determine the daily used number of the cigarettes 150 included in the usage history information on the basis of prescribed information that the user terminal 200 receives from a prescribed device.

The user terminal 200 receives the prescribed information from the prescribed device through communication with the prescribed device (not illustrated). The prescribed device is a lighter provided with a communication function, for example. The lighter provided with a communication function is provided with a communication interface capable of performing communication conforming to any given wired or wireless communication standard. For example, in the case of wireless communication, Wi-Fi(R), Bluetooth(R), or the like may be adopted. The lighter provided with a communication function can use the communication interface to exchange prescribed information with the user terminal 200.

More specifically, the user terminal 200 receives information indicating that the lighter has been used as the prescribed information from the lighter provided with a communication function. The information indicating that the lighter has been used may be, for example, information indicating that the lighter has been ignited. For example, the user can perform a predetermined action on an ignition mechanism of the lighter and thereby cause a high temperature to be produced momentarily to set fire to, that is, ignite, a liquid or solid fuel stored inside the lighter.

Additionally, if the user causes the ignition device to operate while communication with the user terminal 200 is established, the lighter provided with a communication function transmits information indicating that the lighter has been used to the user terminal 200. The lighter provided with a communication function may also transmit information indicating that the lighter has been used to the user terminal 200 when the lighter is ignited. The lighter provided with a communication function may also transmit information indicating that the lighter has been used to the user terminal 200 if the ignition duration is a prescribed time (such as 5 seconds or 10 seconds, for example) or longer.

Alternatively, the prescribed device may also be the case (that is, the package) of the cigarette 150 provided with a communication function, for example. The case provided with a communication function is provided with a communication interface capable of performing communication conforming to any given wired or wireless communication standard. For example, in the case of wireless communication, Wi-Fi(R), Bluetooth(R), or the like may be adopted. The case provided with a communication function can use the communication interface to exchange prescribed information with the user terminal 200.

More specifically, the user terminal 200 receives information indicating that the cigarette 150 has been retrieved from the case as the prescribed information from the case provided with a communication function. The information indicating that the cigarette 150 has been retrieved from the case may also include, for example, information related to the number of the cigarettes 150 retrieved from the case. For example, the case provided with a communication function transmits information indicating that the cigarette 150 has been retrieved from the case to the user terminal 200 when the cigarette 150 is retrieved from the case. The case provided with a communication function may also transmit information related to the number of the cigarettes 150 retrieved from the case as the information indicating that the cigarette 150 has been retrieved from the case to the user terminal 200 on a predetermined interval (such as one day), for example.

The user terminal 200 associates the prescribed information received from the prescribed device with the time of receiving the prescribed information. For example, if information indicating that the lighter has been used is received from the lighter provided with a communication function, the user terminal 200 associates the information indicating that the lighter has been used with the time of receiving the information. As another example, if information indicating that the cigarette 150 has been retrieved from the case provided with a communication function is received, the user terminal 200 associates the information indicating that the cigarette 150 has been retrieved with the time of receiving the information.

The history determiner 231a of the user terminal 200 determines the daily used number of the cigarettes 150 on the basis of the prescribed information received from the prescribed device. As described above, the time of receiving information is associated with the prescribed information received from the prescribed device, and by aggregating the prescribed information on the basis of the time of receiving the information, the daily used number of the cigarettes 150 included in the usage history information can be determined. Also, in the case of the first embodiment, if the prescribed information received from the prescribed device includes information related to the number of the cigarettes 150, the history determiner 231a of the user terminal 200 may treat the number of the cigarettes 150 as the daily used number of the cigarettes 150. Note that in the case of the second embodiment, the history determiner 331b of the server 300 performs a similar process.

With this arrangement, the user terminal 200 can determine the daily used number of the cigarettes 150 included in the usage history information on the basis of prescribed information that the user terminal 200 receives from a prescribed device through various means. Consequently, the user terminal 200 can determine daily used number of the cigarettes 150 included in the usage history information without having the user input usage information about the cigarette 150 manually.

(2) In the above description, an information processing device, an information processing method, and an information processing system according to several embodiments are described with reference to the drawings. It is understood that the present disclosure may also be carried out as a program that, when executed by a processor, causes the processor to operate as the information processing device and/or execute the information processing method, or may be carried out as a computer-readable storage medium storing the program.

The foregoing describes embodiments of the present disclosure along with modifications and applications thereof, but it should be understood that the above examples are merely for illustrative purposes and do not limit the scope of the present disclosure. It should be understood that changes, additions, improvements, and the like can be made to the embodiments appropriately without departing from the gist and scope of the present disclosure. The scope of the present disclosure should not be limited by any of the embodiments described above, but should be defined only by the claims and their equivalents.

REFERENCE SIGNS LIST

> 11 power supply unit
> 12 cartridge
> 13 flavor-imparting cartridge
> 100 aerosol-generating device
> 112, 112a, 112b sensor
> 115, 115a, 115b communicator
> 116, 116a, 116b controller
> 150 cigarette
> 200 user terminal
> 300 server
> 210a, 210b input receiver
> 211a, 211b acquirer
> 212a, 212b reader
> 220a, 220b communicator
> 230a, 330b goal determiner
> 231a, 331b history determiner
> 232a, 332b progress determiner
> 240a, 240b output unit
> 250 application
> 310a, 310b log storage
> 320a, 320b communicator
> 333b ranking determiner
> 400, 500 network
> D10 menu screen
> D20, D20a, D20b goal setting screen
> D30 goal confirmation screen

D40, D40a, D40b history input screen
D50, D50a, D50b progress screen

**Claims**

1. An information processing device comprising:

   an input receiver that receives input of goal information for transitioning from a use of a first inhalation product to a use of a second inhalation product and input of usage information about the first inhalation product;
   an acquirer that acquires usage information about the second inhalation product from the second inhalation product;
   a goal determiner that determines a goal value for each predetermined period on a basis of the goal information;
   a history determiner that determines usage history information for each predetermined period on a basis of the usage information about the first inhalation product and the usage information about the second inhalation product;
   a progress determiner that determines progress information for each predetermined period with respect to the goal value on a basis of the goal value and the usage history information; and
   an output unit that creates and outputs a progress screen on a basis of the progress information.

2. The information processing device according to claim 1, wherein

   the input receiver is further configured to receive input for editing the goal information, and
   the goal determiner is further configured to update the determined goal value on a basis of the edited goal information.

3. The information processing device according to claim 1 or 2, wherein

   the input receiver is further configured to receive the input of usage information about the first inhalation product through a prescribed user interface image every time a user uses the first inhalation product, and
   the usage information about the first inhalation product is associated with a time at which the usage information about the first inhalation product is received.

4. The information processing device according to any one of claims 1 to 3, further comprising:

   a reader for reading a prescribed code, wherein
   the usage information about the first inhalation product includes a brand selected from among a plurality of brands applicable to the first inhalation product, and
   the brand is specified by causing the reader to read the code included in the first inhalation product.

5. The information processing device according to any one of claims 1 to 4, wherein

   the goal information includes a deadline, and
   the goal determiner is configured to determine an ultimate goal value on the deadline on a basis of the goal information and determine the goal value for each predetermined period until the deadline on a basis of the ultimate goal value.

6. The information processing device according to claim 5, wherein

   the predetermined period is set to one day, and
   the daily goal value includes at least one of a daily planned usage value of the first inhalation product or a daily planned usage value of the second inhalation product calculated on a basis of the ultimate goal value and the number of days until the day of the deadline.

7. The information processing device according to claim 6, wherein

   the progress information includes a daily score,
   the progress determiner is configured to determine a daily usage ratio of the second inhalation product on a basis of the usage history information and calculate the daily score on a basis of a ratio of the daily planned usage value of the second inhalation product and the daily usage ratio of the second inhalation product, and the progress screen includes a display of the score.

8. The information processing device according to claim 7, wherein

the progress information includes a daily achievement result ranked on a basis of the daily score, and the progress screen includes a display of a calendar with which the achievement result is visually associated.

9. The information processing device according to any one of claims 6 to 8, wherein

the progress information includes a value of a prescribed indicator calculated on a basis of at least one of the usage information about the first inhalation product and the usage information about the second inhalation product, and the progress screen includes a display of a graph illustrating how the value of the prescribed indicator changes daily over time.

10. The information processing device according to any one of claims 1 to 9, wherein

the first inhalation product is a cigarette, and the second inhalation product is an aerosol-generating device that can communicate with the information processing device.

11. An information processing method comprising:

receiving input of goal information for transitioning from a use of a first inhalation product to a use of a second inhalation product;
determining a goal value for each predetermined period until a deadline on a basis of the goal information;
receiving input of usage information about the first inhalation product;
acquiring usage information about the second inhalation product from the second inhalation product;
determining usage history information for each predetermined period on a basis of the usage information about the first inhalation product and the usage information about the second inhalation product;
determining progress information for each predetermined period with respect to the goal value on a basis of the goal value and the usage history information; and
creating and outputting a progress screen based on the progress information.

12. The information processing method according to claim 11, further comprising:

receiving input for editing the goal information; and
updating the goal value on a basis of the edited goal information.

13. The information processing method according to claim 11 or 12, wherein

the usage information about the first inhalation product is configured to be inputted through a prescribed user interface image every time a user uses the first product, and
the usage information about the second inhalation product is configured to be acquired from the second inhalation product through communication.

14. The information processing method according to any one of claims 11 to 13, wherein

the goal information includes the deadline, and
the determining the goal value includes:

determining an ultimate goal value on the deadline on a basis of the goal information; and
determining the goal value for each predetermined period until the deadline on a basis of the ultimate goal value.

15. The information processing method according to claim 14, wherein

the predetermined period is set to one day, and

the goal value includes at least one of a daily planned usage value of the first inhalation product or a daily planned usage value of the second inhalation product calculated on a basis of the ultimate goal value and the number of days until the day of the deadline.

16. The information processing method according to claim 15, wherein

the progress information includes a daily score calculated on a basis of a ratio of a daily planned usage value of the second inhalation product and a daily usage ratio of the second inhalation product, and

the progress screen includes a display of the score.

17. The information processing method according to claim 16, wherein

the progress information includes a daily achievement result ranked on a basis of the daily score, and

the progress screen includes a display of a calendar with which the achievement result is visually associated.

18. The information processing method according to any one of claims 15 to 17, wherein

the progress information includes a value of a prescribed indicator calculated on a basis of at least one of the usage information about the first inhalation product and the usage information about the second inhalation product, and

the progress screen includes a display of a graph illustrating how the value of the prescribed indicator changes daily over time.

19. An information processing system comprising a user terminal and a server,

the user terminal including:

an input receiver that receives input of goal information to transition from a use of a first inhalation product to a use of a second inhalation product and input of usage information about the first inhalation product;

an acquirer that acquires usage information about the second inhalation product from the second inhalation product;

an output unit that generates and outputs a progress screen based on progress information,

the server including:

a goal determiner that determines a goal value for each predetermined period on a basis of the goal information;

a history determiner that determines usage history information for each predetermined period on a basis of the usage information about the first inhalation product and the usage information about the second inhalation product;

a progress determiner that determines the progress information for each predetermined period with respect to the goal value on a basis of the goal value and the usage history information.

20. The information processing system according to claim 19, wherein

the server further includes a ranking determiner that determines a ranking of the user on a basis of the progress information, and

the progress screen to be displayed by the output unit of the user terminal is configured to display the ranking.

# Fig. 1

# Fig. 2

EP 4 260 740 A1

# Fig. 3

Fig. 4

300

| 31 | 32 | 33 |
|---|---|---|
| PROCESSOR | MEMORY | STORAGE |

| 34 | 35 |
|---|---|
| INPUT-OUTPUT INTERFACE | COMMUNICATION INTERFACE |

39

# Fig. 5

EP 4 260 740 A1

# Fig. 6

AEROSOL-GENERATING DEVICE — 100
USER TERMINAL — 200
SERVER — 300

**USER TERMINAL**

S21a — RECEIVE INPUT OF GOAL INFORMATION

S22a — DETERMINE DAILY GOAL VALUE UNTIL DEADLINE

(1. APPLICATION SETTING)

**AEROSOL-GENERATING DEVICE**

S11a — SPECIFY PUFF COUNT OF AEROSOL-GENERATING DEVICE AND/OR CONSUMPTION QUANTITY OF FLAVOR-IMPARTING CARTRIDGE

S12a — TRANSMIT USAGE INFORMATION TO USER TERMINAL

S23a — RECEIVE INPUT OF USAGE INFORMATION ABOUT CIGARETTE

S24a — ACQUIRE USAGE INFORMATION ABOUT AEROSOL-GENERATING DEVICE

(2. USAGE HISTORY ACQUISITION: REPEAT DURING TRANSITION PERIOD)

S25a — DETERMINE DAILY USAGE HISTORY INFORMATION

S26a — DETERMINE DAILY PROGRESS INFORMATION

S27a — GENERATE AND OUTPUT PROGRESS SCREEN

S28a — TRANSMIT LOG DATA

(3. PROGRESS MANAGEMENT: REPEAT DURING TRANSITION PERIOD)

S31a — STORE LOG DATA

EP 4 260 740 A1

# Fig. 7

FROM S21a

S22a

SPECIFY DEADLINE AND USAGE RATIO OF AEROSOL-GENERATING DEVICE ON DEADLINE FROM GOAL INFORMATION — S221a

DETERMINE ULTIMATE GOAL VALUE ON DEADLINE ON BASIS OF GOAL INFORMATION — S222a

DETERMINE DAILY GOAL VALUE UNTIL DEADLINE DAY ON BASIS OF ULTIMATE GOAL VALUE AND NUMBER OF DAYS UNTIL DEADLINE — S223a

GOAL INFO EDITED? — S224a

Yes

No

TO S23a

Fig. 8

```
                    ┌──────────────────┐  D10
                    │                  │
                    │   MENU SCREEN    │
                    │                  │
                    └──────────────────┘
                             ▲
          ┌──────────┬───────┴───────┬──────────┐
          ▼          ▼               ▼          ▼
   D20         D30              D40         D50
┌─────────┐ ┌─────────────┐ ┌──────────────┐ ┌──────────┐
│  GOAL   │ │    GOAL     │ │HISTORY INPUT │ │ PROGRESS │
│ SETTING │ │CONFIRMATION │ │   SCREEN     │ │  SCREEN  │
│ SCREEN  │ │   SCREEN    │ │ (CIGARETTE)  │ │          │
│         │ │   (DAILY)   │ │              │ │          │
└─────────┘ └─────────────┘ └──────────────┘ └──────────┘
```

EP 4 260 740 A1

# Fig. 9A

INPUT GOAL FOR
TRANSITIONING TO AEROSOL-
GENERATING DEVICE

CURRENT TOTAL OF CIGARETTES
AND AEROSOL-GENERATING DEVICE:

10 ▼

I WILL USE THE AEROSOL-
GENERATING DEVICE, BY

yyyy/mm/dd

% OR MORE

REGISTER

D20a

i22

i20

i21

# Fig. 9B

D20b

SELECT GOAL PLAN FOR
TRANSITIONING TO AEROSOL-
GENERATING DEVICE

CURRENT TOTAL OF CIGARETTES AND
AEROSOL-GENERATING DEVICE:

i25 — | 10 ▼ |

SELECT PLAN:

i26 — ● I MAINLY SMOKE CIGARETTES BUT I WANT TO
INCREASE MY RATIO OF THE AEROSOL-
GENERATING DEVICE

○ I WANT MY USAGE RATIO OF CIGARETTES AND
THE AEROSOL-GENERATING DEVICE TO BE
ABOUT THE SAME

○ I WANT TO USE ONLY THE AEROSOL-
GENERATING DEVICE AND CONTROL HOW MUCH
I USE

GOAL DEADLINE:

i27 — | 2020/4/23 ▼ |

( REGISTER )

Fig. 10

2020/07/22 (WED)

TODAY'S GOAL

TOTAL NUMBER    PUFF COUNT

10              150

D30

i30

i31

PLANNED AMOUNT OF
AEROSOL-GENERATING    7
DEVICE TO USE

i32

PLANNED AMOUNT OF
CIGARETTES TO USE    3

i33

# Fig. 11A

D40a

PRESS WHEN YOU
SMOKE A
CIGARETTE

i40

SMOKED

SELECT BRAND

i41

SMOKING LOG (LATEST 3 ENTRIES)

i42

| yyyymmdd hh:mm | 1 | EDIT | REMOVE |
| yyyymmdd hh:mm | 1 | EDIT | REMOVE |
| yyyymmdd hh:mm | 1 | CONFIRM | BACK |

# Fig. 11B

# Fig. 12A

Daily  **Weekly**  Monthly

< 2020 2/14~2/18 >

PHYSICAL EFFECT INDICATOR

i51

2/14 2/15 2/16 2/17 2/18
× × × ✓ ✓
▨ CIGARETTES ▨ AEROSOL-GENERATING DEVICE

✓ DATA LAST UPDATED: yyyy/mm/dd xx:xx

i52

TRANSITION GOAL ACHIEVEMENT SCORE  72

i53

TODAY'S HISTORY DATA

i55

NUMBER SMOKED  PUFF COUNT

18  82  MANUAL INPUT

i54

i56

D50a

# Fig. 12B

ADVICE:
YOU'RE MAKING GOOD PROGRESS ON WEEKDAYS. KEEP IT UP ON THE WEEKENDS, TOO!

D50b

i58

< APRIL 2020 >

| MON | TUE | WED | THU | FRI | SAT | SUN |
|---|---|---|---|---|---|---|

i57

EXPLANATION OF CALENDAR MARKS

○ SCORE RANGE 80-100%
△ SCORE RANGE 50-79%
✕ SCORE RANGE 0-49%

Fig. 13

EP 4 260 740 A1

# Fig. 14

# Fig. 15

THIS WEEK'S RANKING
(2020/04/05~2020/04/11)
SMOKING MODERATION

① PERSON ●●  XX POINTS

② PERSON ○○  XX POINTS

③ PERSON ○○  XX POINTS

4  PERSON ○○  XX POINTS

5  PERSON ○○  XX POINTS

6  PERSON ○○  XX POINTS

7  PERSON ○○  XX POINTS

D60

## INTERNATIONAL SEARCH REPORT

International application No.

PCT/JP2020/046516

### A. CLASSIFICATION OF SUBJECT MATTER

A24F 40/65(2020.01)i
FI: A24F40/65

According to International Patent Classification (IPC) or to both national classification and IPC

### B. FIELDS SEARCHED

Minimum documentation searched (classification system followed by classification symbols)
A24F40/65

Documentation searched other than minimum documentation to the extent that such documents are included in the fields searched

| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2020-502992 A (PHILIP MORRIS PRODUCTS S.A.) 30 January 2020 (2020-01-30) paragraphs [0078]- | 1-4, 10-13, 19-20 |
| A | [0089], fig. 1-6 | 5-9, 14-18 |
| Y | US 2019/0183185 A1 (DR. REDDY'S LABORATORIES LIMITED) 20 June 2019 (2019-06-20) fig. 6, | 1-4, 10-13, 19-20 |
| A | paragraphs [0115], [0124], [0128]-[0129] | 5-9, 14-18 |
| Y | US 2020/0035118 A1 (PANDOLFINO, Joseph) 30 January 2020 (2020-01-30) fig. 1-3, paragraphs [0140]- | 1-4, 10-13, 19-20 |
| A | [0154] | 5-9, 14-18 |
| A | US 2016/0278435 A1 (SMOKEWATCHERS SAS) 29 September 2016 (2016-09-29) fig. 1-7 | 1-20 |
| A | US 2004/0031498 A1 (BRUE, Vesta L.) 19 February 2004 (2004-02-19) fig. 5-8 | 1-20 |

☐ Further documents are listed in the continuation of Box C.  ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 06 January 2021 (06.01.2021) | 19 January 2021 (19.01.2021) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**
Information on patent family members

| International application No. |
|---|
| PCT/JP2020/046516 |

| Patent Documents referred in the Report | Publication Date | Patent Family | Publication Date |
|---|---|---|---|
| JP 2020-502992 A | 30 Jan. 2020 | US 2019/0221130 A1 fig. 1-6, paragraphs [0083]-[0094] WO 2018/060798 A1 CN 109690658 A KR 10-2019-0051964 A | |
| US 2019/0183185 A1 | 20 Jun. 2019 | WO 2018/025217 A1 | |
| US 2020/0035118 A1 | 30 Jan. 2020 | WO 2020/023848 A1 | |
| US 2016/0278435 A1 | 29 Sep. 2016 | WO 2015/063126 A1 CN 106535673 A | |
| US 2004/0031498 A1 | 19 Feb. 2004 | WO 2003/001479 A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• WO 2018060798 A